# EUROPEAN PATENT APPLICATION

(11) **EP 4 559 916 A1**
(43) Date of publication of application: **28.05.2025**
(21) Application number: 23842427.9
(22) Date of filing: 21.07.2023
(51) Int. Cl.: C07D 487/04, A61K 31/519, A61P 35/00

(54) **PHARMACEUTICALLY ACCEPTABLE SALT AND POLYMORPH OF SUBSTITUTED PYRAZOLO[1,5-A]PYRIMIDIN-7-AMINE DERIVATIVE AND USE THEREOF**

(30) Priority: 22.07.2022 CN 202210866135; 22.07.2022 CN 202210870337
(71) Applicant: Shanghai Haiyan Pharmaceutical Technology Co., Ltd., Shanghai 201203 (CN); Yangtze River Pharmaceutical Group Co., Ltd., Taizhou, Jiangsu 225321 (CN)
(72) Inventor: DAN, Zhaoling, Shanghai 201203 (CN); JIANG, Taotao, Shanghai 201203 (CN); WANG, Jibiao, Shanghai 201203 (CN); XUE, Yuan, Shanghai 201203 (CN)
(74) Representative: Staeger & Sperling Partnerschaftsgesellschaft mbB
(86) International application number: PCT/CN2023/108554
(87) International publication number: WO 2024/017365

(57) **Abstract**

The present disclosure relates to a pharmaceutically acceptable salt and polymorph of a substituted pyrazolo[1,5-a]pyrimidin-7-amine derivative as represented by Formula (I), a pharmaceutical composition comprising the same, a preparation method therefor, and use of the pharmaceutically acceptable salt and the polymorph in treating or preventing diseases related to CDK9 activity or mediated by CDK9 activity.

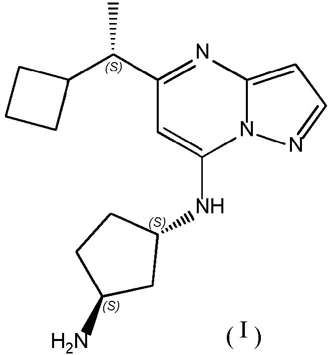

## Description

### RELATED APPLICATION

The present application claims the priority of the Chinese Patent Application No. 202210866135.0, with the title of "PHARMACEUTICALLY ACCEPTABLE SALT AND POLYMORPH OF SUBSTITUTED PYRAZOLO[1,5-A]PYRIMIDIN-7-AMINE DERIVATIVE AND USE THEREOF", and Application No. 202210870337.2, with the title of "PHOSPHATE SALT AND POLYMORPH OF CDK9 INHIBITOR AND USE THEREOF", filed on July 22, 2022 before the China National Intellectual Property Administration, which are incorporated herein by reference in their entirety.

### TECHNICAL FIELD

The present disclosure belongs to the field of medical technology, in particular relates to a pharmaceutically acceptable salt and polymorph of a substituted pyrazolo[1,5-a]pyrimidin-7-amine derivative, a pharmaceutical composition comprising the same, a preparation method therefor, and medical use thereof.

### BACKGROUND

CDK9 is a member of cell cyclin-dependent kinase (CDK) protein family and plays an important role in gene transcription regulation. CDK9 mainly regulates transcription elongation of genes by phosphorylating the carboxy-terminal domain of RNA polymerase II. CDK9 is generally highly expressed in tumors and is also an important factor in progression and maintenance of tumor cells. A CDK9 inhibitor down-regulates the expression of relevant oncoprotein (MYC) and the expression of antiapoptotic protein Mcl-1 by inhibiting the transcriptional elongation of genes, thereby promoting apoptosis of cancer cells. The CDK9 inhibitor reactivates a silent gene by regulating epigenetic factor BRG1, including the activation of endogenous retroviruses (ERVs) in tumor cells, promotes interferon expression, and makes tumor cells more sensitive to immunotherapy.

At present, many companies are engaged in the development of CDK9 inhibitors, including the selective CDK9 inhibitor BAY1251152 developed by Bayer, the selective CDK9 inhibitor AZD4573 developed by AstraZeneca, the non-selective CDK9 inhibitor TP-1287 developed by Tolero, and the non-selective CDK9 inhibitor QHRD107 developed by Changzhou Qianhong Pharmaceutical Company, and the like.

The solubility and stability of active ingredients in pharmaceuticals have an important impact on the research of druggability, and the solid form is beneficial to the quality control of a product. Therefore, it is necessary to explore the solid form of the product.

### SUMMARY

Based on this, the object of the present disclosure is to provide a pharmaceutically acceptable salt and polymorph of a substituted pyrazolo[1,5-a]pyrimidin-7-amine derivative and use thereof. Specifically, the pharmaceutically acceptable salt is an L-tartrate and a phosphate of the substituted pyrazolo[1,5-a]pyrimidin-7-amine derivative. The substituted pyrazolo[1,5-a]pyrimidin-7-amine derivative is a CDK9 inhibitor and has a molecular structure of (1S,3S)-N1-(5-((S)-1-cyclobutylethyl)pyrazolo[1,5-a]pyrimidin-7-yl)cyclopentan-1,3-diamine as shown in Formula (I).

A first aspect of the present disclosure provides a pharmaceutically acceptable salt of a compound of Formula (I): 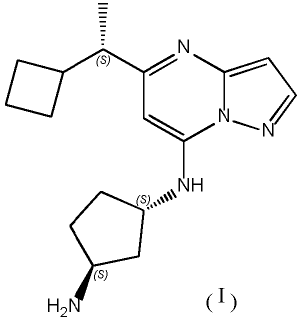 wherein, the pharmaceutically acceptable salt is selected from the group consisting of a phosphate and an L-tartrate.

In some embodiments, the pharmaceutically acceptable salt of the compound of Formula (I) is in an anhydrous form, a hydrate form, or a solvate form.

In some embodiments, the pharmaceutically acceptable salt of the compound of Formula (I) is a crystal. In some embodiments, the pharmaceutically acceptable salt of the compound of Formula (I) is amorphous.

In some embodiments, the pharmaceutically acceptable salt is a phosphate. In some embodiments, the phosphate is a first type of phosphate of the compound of Formula (I), wherein a molar ratio of phosphoric acid to the compound of Formula (I) is (1.8-2.4):1. In some embodiments, the molar ratio of phosphoric acid to the compound of Formula (I) can be 1.8:1, 1.9:1, 2.0:1, 2.1:1, 2.2:1, 2.3:1 or 2.4:1. In some embodiments, the molar ratio of phosphoric acid to the compound of Formula (I) is (1.9-2.3):1. In some embodiments, the molar ratio of phosphoric acid to the compound of Formula (I) is 2:1.

In some embodiments, the phosphate is a phosphate crystal form I of the compound of Formula (I), an X-ray powder diffraction pattern of which has characteristic diffraction peaks at the diffraction angle 2θ (°) values of 18.234±0.2, 19.131±0.2 and 21.266±0.2. In some embodiments, the phosphate is a phosphate crystal form I of the compound of Formula (I), the X-ray powder diffraction pattern of which has characteristic diffraction peaks at the diffraction angle 2θ (°) values of 18.803± 0.2, 18.234±0.2, 19.131±0.2 and 21.266±0.2.

In some embodiments, the X-ray powder diffraction pattern of the phosphate crystal form I further includes characteristic diffraction peaks at 2 or more (such as 3, 4, 5, 6, 7, 8, 9, 10, 11, 12, 13, 14, 15, etc.) diffraction angle 2θ (°) values selected from the group consisting of 2.121±0.2, 7.373±0.2, 9.556±0.2, 10.607±0.2, 11.105±0.2, 12.322±0.2, 12.917±0.2, 13.999±0.2, 14.902±0.2, 15.238±0.2, 18.803±0.2, 19.881±0.2, 20.697±0.2, 22.01±0.2, 22.464±0.2, 23.247±0.2, 24.025±0.2, 26.367±0.2, 28.513±0.2, 30.297±0.2, 31.216±0.2 and 34.017±0.2, in addition to characteristic diffraction peaks at the diffraction angle 2θ (°) values of 18.234±0.2, 19.131±0.2 and 21.266±0.2.

In some embodiments, the X-ray powder diffraction pattern of the phosphate crystal form I has characteristic diffraction peaks at 3 or more (such as 3, 4, 5, 6, 7, 8, 9, 10, 11, 12, 13, 14, 15, etc.) or all diffraction angle 2θ (°) values selected from the group consisting of 2.121±0.2, 7.373±0.2, 9.556±0.2, 10.607±0.2, 11.105±0.2, 12.322±0.2, 12.917±0.2, 13.999±0.2, 14.902±0.2, 15.238±0.2, 18.234±0.2, 18.803±0.2, 19.131±0.2, 19.881±0.2, 20.697±0.2, 21.266±0.2, 22.01±0.2, 22.464±0.2, 23.247±0.2, 24.025±0.2, 26.367±0.2, 28.513±0.2, 30.297±0.2, 31.216±0.2 and 34.017±0.2.

In some embodiments, the X-ray powder diffraction pattern of the phosphate crystal form I has characteristic diffraction peaks at the diffraction angle 2θ (°) values of 13.999±0.2, 18.234±0.2, 18.803±0.2, 19.131±0.2, 21.266±0.2, 22.01±0.2 and 23.247±0.2.

In some embodiments, the X-ray powder diffraction pattern of the phosphate crystal form I has characteristic diffraction peaks at the diffraction angle 2θ (°) values of 7.373±0.2, 10.607±0.2, 11.105±0.2, 12.322±0.2, 12.917±0.2, 13.999±0.2, 14.902±0.2, 15.238±0.2, 18.234±0.2, 18.803±0.2, 19.131±0.2, 20.697±0.2, 21.266±0.2, 22.01±0.2, 22.464±0.2, 23.247±0.2, 24.025±0.2, 26.367±0.2 and 28.513±0.2.

In some embodiments, the X-ray powder diffraction pattern of the phosphate crystal form I has characteristic diffraction peaks at the diffraction angle 2θ (°) values of 2.121±0.2, 7.373±0.2, 9.556±0.2, 10.607±0.2, 11.105±0.2, 12.322±0.2, 12.917±0.2, 13.999±0.2, 14.902±0.2, 15.238±0.2, 18.234±0.2, 18.803±0.2, 19.131±0.2, 19.881±0.2, 20.697±0.2, 21.266±0.2, 22.01±0.2, 22.464±0.2, 23.247±0.2, 24.025±0.2, 26.367±0.2, 28.513±0.2, 30.297±0.2, 31.216±0.2 and 34.017±0.2.

In some embodiments, the X-ray powder diffraction pattern of the phosphate crystal form I of the compound of Formula (I) has characteristic diffraction peaks expressed in 2θ (°) values and d values as shown in Table 1, and the relative intensity of each peak is shown in Table 1.

**Table 1. 2θ (°), d values and relative intensity I/I₀ of the phosphate crystal form I**

| 2θ (°) (±0.2) | d value (Å) (±0.2) | Relative Intensity I/I₀ (%) | 2θ (°) (±0.2) | d value (Å) (±0.2) | Relative Intensity I/I₀ (%) |
|---|---|---|---|---|---|
| 2.121 | 41.6219 | 7.4 | 19.881 | 4.4623 | 7.9 |
| 7.373 | 11.9804 | 11.7 | 20.697 | 4.2881 | 19.4 |
| 9.556 | 9.2481 | 4.9 | 21.266 | 4.1747 | 67.5 |
| 10.607 | 8.3339 | 20.7 | 22.01 | 4.0352 | 18.7 |
| 11.105 | 7.9611 | 6.2 | 22.464 | 3.9546 | 14.2 |
| 12.322 | 7.1774 | 10.4 | 23.247 | 3.8232 | 39.1 |
| 12.917 | 6.8479 | 16.2 | 24.025 | 3.7012 | 8.7 |
| 13.999 | 6.3209 | 24.9 | 26.367 | 3.3775 | 35 |
| 14.902 | 5.94 | 18.5 | 28.513 | 3.1279 | 15.9 |
| 15.238 | 5.8097 | 24.3 | 30.297 | 2.9477 | 4.1 |
| 18.234 | 4.8614 | 96.4 | 31.216 | 2.863 | 10.5 |
| 18.803 | 4.7155 | 100 | 34.017 | 2.6334 | 8.3 |
| 19.131 | 4.6355 | 54.3 | | | |

In some embodiments, the phosphate crystal form I of the compound of Formula (I) has the molar ratio of phosphoric acid to the compound of Formula (I) of 2:1.

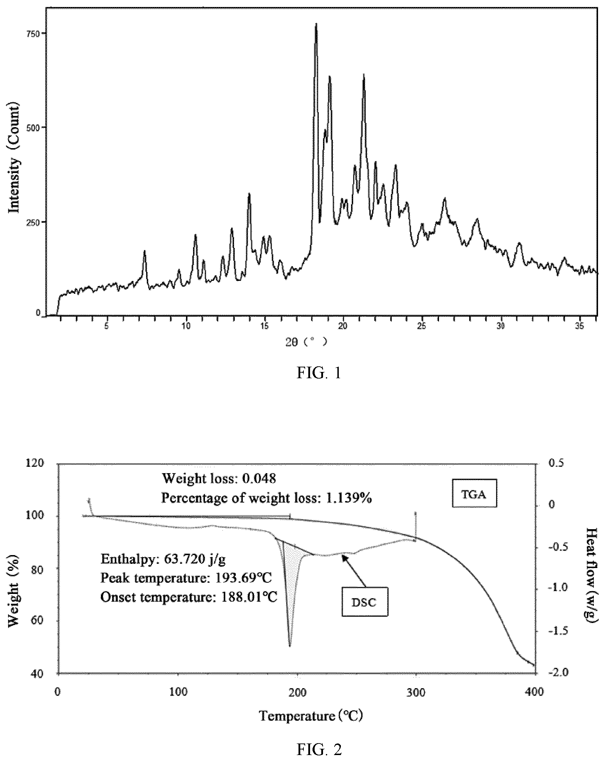

In some embodiments, the X-ray powder diffraction pattern of the phosphate crystal form I of the compound of Formula (I) is substantially as shown in FIG. 1.

In some embodiments, a differential scanning calorimeter curve of the phosphate crystal form I of the compound of Formula (I) has endothermic peaks at 188.01°C±3°C, 188.01°C±2°C, 188.01°C±1°C or 188.01°C±0.5°C. In some embodiments, the differential scanning calorimeter curve of the phosphate crystal form I of the compound of Formula (I) has an onset temperature of 188.01°C±3°C, 188.01°C±2°C, 188.01°C±1°C or 188.01°C±0.5°C, and has a peak temperature of 193.69°C±3°C, 193.69°C±2°C, 193.69°C±1°C or 193.69°C±0.5°C. In some embodiments, a differential scanning calorimetry spectrum (DSC spectrum) of the phosphate crystal form I of the compound of Formula (I) is substantially as shown in FIG. 2. In the embodiment as shown in FIG. 2, the phosphate salt form I of the compound of Formula (I) has a melting point of about 188.01±0.5°C.

In some embodiments, a thermogravimetric analysis spectrum (TGA spectrum) of the phosphate crystal form I of the compound of Formula (I) is substantially as shown in FIG. 2. In some embodiments, the TGA spectrum of the phosphate crystal form I of the compound of Formula (I) shows a weight loss of 1.139% at around 190°C.

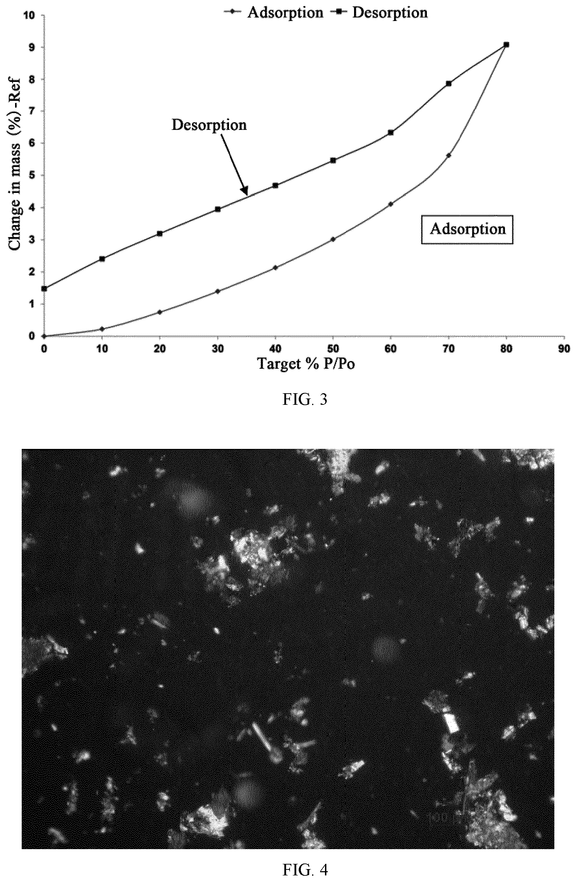

In some embodiments, a dynamic vapor sorption spectrum (DVS spectrum) of the phosphate crystal form I of the compound of Formula (I) is substantially as shown in FIG. 3. In some embodiments, the DVS spectrum of the phosphate crystal form I of the compound of Formula (I) shows a moisture absorption weight gain of 9% at 80% relative humidity (RH).

In some embodiments, a microscope image of the phosphate crystal form I of the compound of Formula (I) is substantially as shown in FIG. 4. In some embodiments, the microscope image of the phosphate crystal form I of the compound of Formula (I) shows the phosphate crystal form I is rod-shaped and block-shaped.

In the first aspect, in some embodiments, the pharmaceutically acceptable salt is an L-tartrate. A molar ratio of L-tartaric acid to the compound of Formula (I) in the L-tartrate is (0.8-1.2):1. In some embodiments, the molar ratio of L-tartaric acid to the compound of Formula (I) can be 0.8:1, 0.9:1, 1.0:1, 1.1:1 or 1.2:1. In some embodiments, the molar ratio of L-tartaric acid to the compound of Formula (I) is (0.9-1.1):1. In some embodiments, the molar ratio of L-tartaric acid to the compound of Formula (I) is 1:1.

In some embodiments, the L-tartrate is an L-tartrate crystal form I of the compound of Formula (I), an X-ray powder diffraction pattern of which has characteristic diffraction peaks at the diffraction angle 2θ (°) values of 13.946±0.2, 16.881±0.2, 19.405±0.2, 21.505±0.2 and 24.262±0.2.

In some embodiments, the X-ray powder diffraction pattern of the phosphate crystal form I further includes characteristic diffraction peaks at 2 or more (such as 3, 4, 5, 6, 7, 8, 9, 10, 11, 12, 13, 14, 15, etc.) diffraction angle 2θ (o) values selected from the group consisting of 6.687±0.2, 7.436±0.2, 9.493±0.2, 10.615±0.2, 12.053±0.2, 12.776±0.2, 13.164±0.2, 14.875±0.2, 15.201±0.2, 16.013±0.2, 18.175±0.2, 19.045±0.2, 20.659±0.2, 22.434±0.2, 23.04±0.2, 25.202±0.2, 26.452±0.2, 28.105±0.2, 29.692±0.2, 31.579±0.2, 34.139±0.2 and 34.543±0.2, in addition to characteristic diffraction peaks at the diffraction angle 2θ (o) values of 13.946±0.2, 16.881±0.2, 19.405±0.2, 21.505±0.2 and 24.262±0.2.

In some embodiments, the X-ray powder diffraction pattern of the L-tartrate crystal form I has characteristic diffraction peaks at 5 or more (such as 5, 6, 7, 8, 9, 10, 11, 12, 13, 14, 15, etc.) or all diffraction angle 2θ (°) values selected from the group consisting of 6.687±0.2, 7.436±0.2, 9.493±0.2, 10.615±0.2, 12.053±0.2, 12.776±0.2, 13.164±0.2, 13.946±0.2, 14.875±0.2, 15.201±0.2, 16.013±0.2, 16.881±0.2, 18.175±0.2, 19.045±0.2, 19.405±0.2, 20.659±0.2, 21.505±0.2, 22.434±0.2, 23.04±0.2, 24.262±0.2, 25.202±0.2, 26.452±0.2, 28.105±0.2, 29.692±0.2, 31.579±0.2, 34.139±0.2 and 34.543±0.2.

In some embodiments, the X-ray powder diffraction pattern of the L-tartrate crystal form I has characteristic diffraction peaks at the diffraction angle 2θ (°) values of 7.436±0.2, 13.946±0.2, 16.013±0.2, 16.881±0.2, 18.175±0.2, 19.045±0.2, 19.405±0.2, 21.505±0.2, 24.262±0.2, 25.202±0.2, 26.452±0.2, 28.105±0.2 and 31.579±0.2.

In some embodiments, the X-ray powder diffraction pattern of the L-tartrate crystal form I has characteristic diffraction peaks at the diffraction angle 2θ (°) values of 6.687±0.2, 7.436±0.2, 10.615±0.2, 12.053±0.2, 13.164±0.2, 13.946±0.2, 14.875±0.2, 15.201±0.2, 16.013±0.2, 16.881±0.2, 18.175±0.2, 19.045±0.2, 19.405±0.2, 20.659±0.2, 21.505±0.2, 22.434±0.2, 23.04±0.2, 24.262±0.2, 25.202±0.2, 26.452±0.2, 28.105±0.2, 29.692±0.2, 31.579±0.2, 34.139±0.2 and 34.543±0.2.

In some embodiments, the X-ray powder diffraction pattern of the L-tartrate crystal form I has characteristic diffraction peaks at the diffraction angle 2θ (°) values of 6.687±0.2, 7.436±0.2, 9.493±0.2, 10.615±0.2, 12.053±0.2, 12.776±0.2, 13.164±0.2, 13.946±0.2, 14.875±0.2, 15.201±0.2, 16.013±0.2, 16.881±0.2, 18.175±0.2, 19.045±0.2, 19. 405±0.2, 20.659±0.2, 21.505±0.2, 22.434±0.2, 23.04±0.2, 24.262±0.2, 25.202±0.2, 26.452±0.2, 28.105±0.2, 29.692±0.2, 31.579±0.2, 34.139±0.2 and 34.543±0.2.

In some embodiments, the X-ray powder diffraction pattern of the L-tartrate crystal form I of the compound of Formula (I) has characteristic diffraction peaks expressed in 2θ (°) values and d values as shown in Table 2, and the relative intensity of each peak is shown in Table 2.

**Table 2. 2θ (°), d values and relative intensity I/I₀ of the L-tartrate crystal form I**

| 2θ (°) (±0.2) | d value (Å) (±0.2) | Relative Intensity I/I₀ (%) | 2θ (°) (±0.2) | d value (Å) (±0.2) | Relative Intensity I/I₀ (%) |
|---|---|---|---|---|---|
| 2.062 | 42.8181 | 13.2 | 19.045 | 4.6562 | 49.8 |
| 6.687 | 13.207 | 6.6 | 19.405 | 4.5707 | 63 |
| 7.436 | 11.8794 | 24.3 | 20.659 | 4.2959 | 12.8 |
| 9.493 | 9.3093 | 5.4 | 21.505 | 4.1288 | 87.9 |
| 10.615 | 8.3278 | 9.4 | 22.434 | 3.9598 | 5.5 |
| 12.053 | 7.3372 | 15.7 | 23.04 | 3.8571 | 16 |
| 12.776 | 6.9234 | 6.1 | 24.262 | 3.6655 | 100 |
| 13.164 | 6.7199 | 12.9 | 25.202 | 3.5309 | 21.6 |
| 13.946 | 6.3452 | 62.9 | 26.452 | 3.3668 | 47 |
| 14.875 | 5.9508 | 12.4 | 28.105 | 3.1724 | 27.4 |
| 15.201 | 5.8241 | 12.6 | 29.692 | 3.0063 | 25.9 |
| 16.013 | 5.5303 | 33.4 | 31.579 | 2.8309 | 46.6 |
| 16.881 | 5.2479 | 49 | 34.139 | 2.6243 | 27.8 |
| 18.175 | 4.8772 | 45.3 | 34.543 | 2.5944 | 18.6 |

In some embodiments, the L-tartrate crystal form I has the molar ratio of L-tartaric acid to the compound of Formula (I) of 1:1.

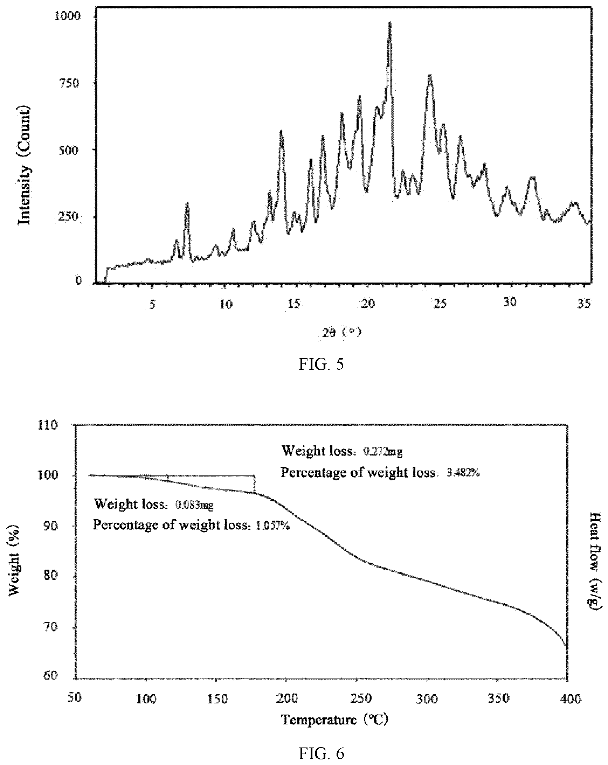

In some embodiments, the X-ray powder diffraction pattern of the L-tartrate crystal form I of the compound of Formula (I) is substantially as shown in FIG. 5.

In some embodiments, a thermogravimetric analysis spectrum of the L-tartrate crystal form I of the compound of Formula (I) is substantially as shown in FIG. 6. In some embodiments, the TGA spectrum of the L-tartrate crystal form I of the compound of Formula (I) shows a weight loss of 1.057% at around 110°C, and a weight loss of 3.482% at around 178°C.

In some embodiments, the L-tartrate is an L-tartrate crystal form II of the compound of Formula (I), an X-ray powder diffraction pattern of which has characteristic diffraction peaks at the diffraction angle 2θ (°) values of 11.662±0.2 and 21.353±0.2.

In some embodiments, the X-ray powder diffraction pattern of the L-tartrate crystal form II further includes characteristic diffraction peaks at 2 or more (such as 3, 4, 5, 6, 7, 8, 9, 10, 11, 12, 13, 14, etc.) diffraction angle 2θ (°) values selected from the group consisting of 6.985±0.2, 10.376±0.2, 10.884±0.2, 12.994±0.2, 14.244±0.2, 16.548±0.2, 17.481±0.2, 18.349±0.2, 18.984±0.2, 21.026±0.2, 26.925±0.2, 29.335±0.2, 30.896±0.2 and 31.784±0.2, in addition to characteristic diffraction peaks at the diffraction angle 2θ (°) values of 11.662±0.2 and 21.353±0.2.

In some embodiments, the X-ray powder diffraction pattern of the L-tartrate crystal form II has characteristic diffraction peaks at 3 or more (such as 3, 4, 5, 6, 7, 8, 9, 10, 11, 12, 13, 14, 15, etc.) diffraction angle 2θ (°) values selected from the group consisting of 6.985±0.2, 10.376±0.2, 10.884±0.2, 11.662±0.2, 12.994±0.2, 14.244±0.2, 16.548±0.2, 17.481±0.2, 18.349±0.2, 18.984±0.2, 21.026±0.2, 21.353±0.2, 26.925±0.2, 29.335±0.2, 30.896±0.2 and 31.784±0.2.

In some embodiments, the X-ray powder diffraction pattern of the L-tartrate crystal form II has characteristic diffraction peaks at the diffraction angle 2θ (°) values of 11.662±0.2, 14.244±0.2, 17.481±0.2, 18.349±0.2, 21.026±0.2 and 21.353±0.2.

In some embodiments, the X-ray powder diffraction pattern of the L-tartrate crystal form II has characteristic diffraction peaks at the diffraction angle 2θ (°) values of 10.376±0.2, 11.662±0.2, 14.244±0.2, 16.548±0.2, 17.481±0.2, 18.349±0.2, 18.984±0.2, 21.026±0.2, 21.353±0.2, 26.925±0.2, 29.335±0.2 and 31.784±0.2.

In some embodiments, the X-ray powder diffraction pattern of the L-tartrate crystal form II has characteristic diffraction peaks at the diffraction angle 2θ (°) values of 6.985±0.2, 10.376±0.2, 10.884±0.2, 11.662±0.2, 12.994±0.2, 14.244±0.2, 16.548±0.2, 17.481±0.2, 18.349±0.2, 18.984±0.2, 21.026±0.2, 21.353±0.2, 26.925±0.2, 29.335±0.2, 30.896±0.2 and 31.784±0.2.

In some embodiments, the X-ray powder diffraction pattern of the L-tartrate crystal form II has characteristic diffraction peaks expressed in 2θ (°) values and d values as shown in Table 3, and the relative intensity of each peak is shown in Table 3.

**Table 3. 2θ (°), d values and relative intensity I/I₀ of the L-tartrate crystal II**

| 2θ (°) (±0.2) | d value (Å) (±0.2) | Relative Intensity I/I₀ (%) | 2θ (°) (±0.2) | d value (Å) (±0.2) | Relative Intensity I/I₀ (%) |
|---|---|---|---|---|---|
| 2.107 | 41.8968 | 2.8 | 17.481 | 5.0692 | 30.7 |
| 3.563 | 24.7756 | 3.1 | 18.349 | 4.8311 | 16.4 |
| 6.985 | 12.6449 | 1.9 | 18.984 | 4.671 | 12 |
| 10.376 | 8.5185 | 2 | 21.026 | 4.2217 | 62.4 |
| 10.884 | 8.1222 | 4.6 | 21.353 | 4.1578 | 100 |
| 11.662 | 7.5823 | 20.3 | 26.925 | 3.3088 | 15.4 |
| 12.994 | 6.8079 | 1.9 | 29.335 | 3.0421 | 5.9 |
| 14.244 | 6.2131 | 17.6 | 30.896 | 2.8919 | 3.3 |
| 16.548 | 5.3529 | 4.6 | 31.784 | 2.8131 | 6.8 |

In some embodiments, the L-tartrate crystal form II has the molar ratio of L-tartaric acid to the compound of Formula (I) of 1:1.

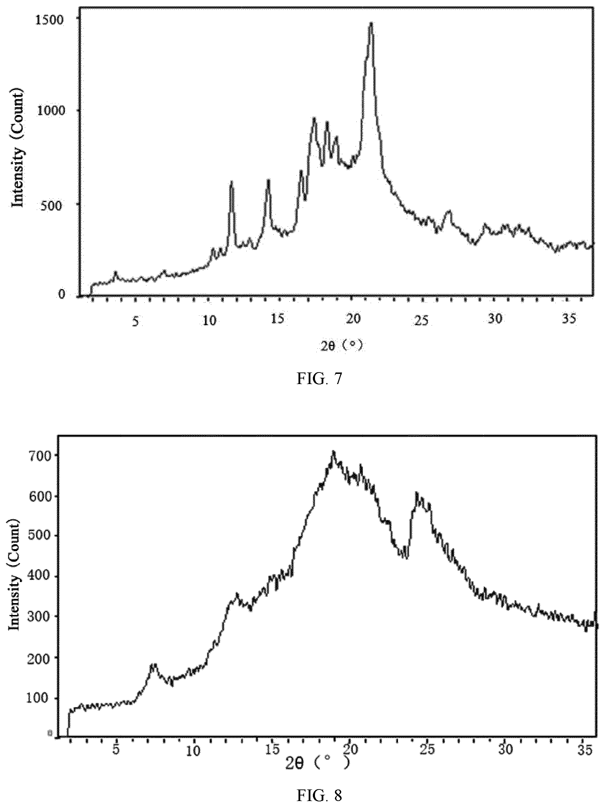

In some embodiments, the X-ray powder diffraction pattern of the L-tartrate crystal form II is substantially as shown in FIG. 7.

In the first aspect, in some embodiments, the phosphate is a second type of phosphate of the compound of Formula (I), wherein a molar ratio of the compound of Formula (I) to phosphoric acid in the phosphate is 1:(0.8-1.2). In some embodiments, the molar ratio of the compound of Formula (I) to phosphoric acid is 1:(0.9-1.1). In some embodiments, the molar ratio of the compound of Formula (I) to phosphoric acid can be 1:0.8, 1:0.9, 1:1, 1:1.1 or 1:1.2. In some embodiments, the molar ratio of the compound of Formula (I) to phosphoric acid in the phosphate is 1:1.

In some embodiments, the phosphate of the compound of Formula (I) is in an anhydrous form, a hydrate form, or a solvate form.

In some embodiments, the phosphate of the compound of Formula (I) is a crystalline powder and exists in crystalline form.

In some embodiments, the phosphate of the compound of Formula (I) is a phosphate crystal form II, an X-ray powder diffraction pattern of which has characteristic diffraction peaks at the diffraction angle 2θ (°) values of 18.230±0.2 and 21.144±0.2.

In some embodiments, the phosphate crystal form II has the molar ratio of the compound of Formula (I) to phosphoric acid of 1:1.

In some embodiments, the X-ray powder diffraction pattern of the phosphate crystal form II further includes characteristic diffraction peaks at 2 or more (such as 2, 3, 4, 5, 6, 7, 8, 9, 10, 11, 12, 13, 14, 15, etc.) diffraction angle 2θ (°) values selected from the group consisting of 10.584±0.2, 11.115±0.2, 11.813±0.2, 12.825±0.2, 13.969±0.2, 14.873±0.2, 15.313±0.2, 18.771±0.2, 19.85±0.2, 20.576±0.2, 22.01±0.2, 22.492±0.2, 23.153±0.2, 23.96±0.2, 24.947±0.2, 26.273±0.2, 27.837±0.2, 28.432±0.2, 30.143±0.2, 31.072±0.2, 31.88±0.2 and 33.982±0.2, in addition to characteristic diffraction peaks at the diffraction angle 2θ (°) values of 18.230±0.2 and 21.144±0.2.

In some embodiments, the X-ray powder diffraction pattern of the phosphate crystal form II has characteristic diffraction peaks at 2 or more (such as 2, 3, 4, 5, 6, 7, 8, 9, 10, etc.) diffraction angle 2θ (°) values selected from the group consisting of 10.584±0.2, 13.969±0.2, 14.873±0.2, 18.230±0.2, 20.576±0.2, 21.144±0.2, 22.01±0.2, 22.492±0.2, 23.153±0.2 and 23.96±0.2.

In some embodiments, the X-ray powder diffraction pattern of the phosphate crystal form II has characteristic diffraction peaks at the diffraction angle 2θ (°) values of 10.584±0.2, 13.969±0.2, 14.873±0.2, 18.230±0.2, 20.576±0.2, 21.144±0.2, 22.01±0.2, 22.492±0.2, 23.153±0.2 and 23.96±0.2.

In some embodiments, the X-ray powder diffraction pattern of the phosphate crystal form II has characteristic diffraction peaks at 2 or more (such as 2, 3, 4, 5, 6, 7, 8, 9, 10, 11, 12, 13, 14, 15, etc.) diffraction angle 2θ (°) values selected from the group consisting of 10.584±0.2, 11.115±0.2, 11.813±0.2, 12.825±0.2, 13.969±0.2, 14.873±0.2, 15.313±0.2, 18.230±0.2, 18.771±0.2, 19.85±0.2, 20.576±0.2, 21.144±0.2, 22.01±0.2, 22.492±0.2, 23.153±0.2, 23.96±0.2, 24.947±0.2, 26.273±0.2, 27.837±0.2, 28.432±0.2, 30.143±0.2, 31.072±0.2, 31.88±0.2 and 33.982±0.2.

In some embodiments, the X-ray powder diffraction pattern of the phosphate crystal form II has characteristic diffraction peaks at the diffraction angle 2θ (°) values of 10.584±0.2, 11.115±0.2, 11.813±0.2, 12.825±0.2, 13.969±0.2, 14.873±0.2, 15.313±0.2, 18.230±0.2, 18.771±0.2, 19.85±0.2, 20.576±0.2, 21.144±0.2, 22.01±0.2, 22.492±0.2, 23.153±0.2, 23.96±0.2, 24.947±0.2, 26.273±0.2, 27.837±0.2, 28.432±0.2, 30.143±0.2, 31.072±0.2, 31.88±0.2 and 33.982±0.2.

In some embodiments, the X-ray powder diffraction pattern of the phosphate crystal form II has characteristic diffraction peaks expressed in 2θ (°) values and d values as shown in Table 4, and the relative intensity of each peak is shown in Table 4.

**Table 4. 2θ (°), d values and relative intensity I/I₀ of the phosphate crystal form II**

| 2θ (°) (±0.2) | d value (Å) (±0.2) | Relative Intensity I/I₀ (%) | 2θ (°) (±0.2) | d value (Å) (±0.2) | Relative Intensity I/I₀ (%) |
|---|---|---|---|---|---|
| 1.973 | 44.7461 | 1 | 21.144 | 4.1984 | 100 |
| 3.687 | 23.9451 | 1.3 | 22.01 | 4.0352 | 5.8 |
| 10.584 | 8.3516 | 12.4 | 22.492 | 3.9498 | 10.8 |
| 11.115 | 7.9543 | 5.7 | 23.153 | 3.8385 | 13.9 |
| 11.813 | 7.4853 | 0.7 | 23.96 | 3.711 | 12.4 |
| 12.825 | 6.8971 | 0.8 | 24.947 | 3.5664 | 8.3 |
| 13.969 | 6.3348 | 10.2 | 26.273 | 3.3893 | 6.3 |
| 14.873 | 5.9516 | 10.4 | 27.837 | 3.2024 | 6.2 |
| 15.313 | 5.7817 | 2.8 | 28.432 | 3.1366 | 14.3 |
| 18.230 | 4.8624 | 73.2 | 30.143 | 2.9624 | 3 |
| 18.771 | 4.7236 | 5.3 | 31.072 | 2.8759 | 5.4 |
| 19.85 | 4.4691 | 4.4 | 31.88 | 2.8049 | 2.5 |
| 20.576 | 4.3132 | 10.1 | 33.982 | 2.636 | 6.8 |

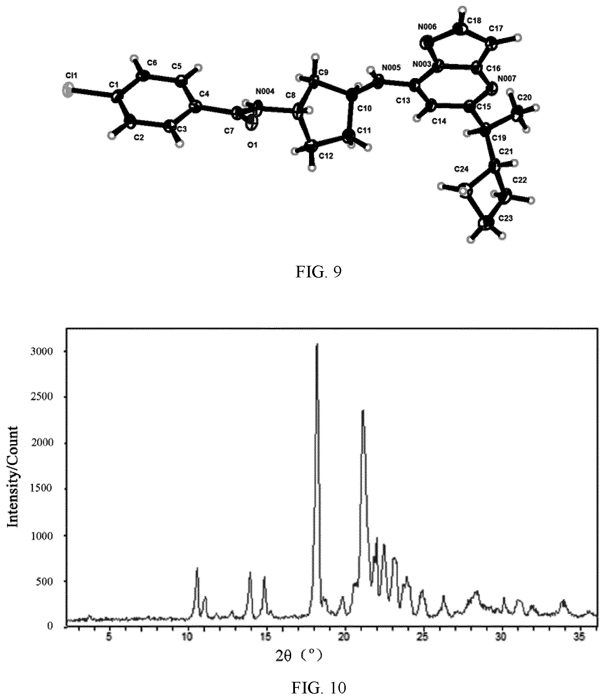

In some embodiments, the X-ray powder diffraction pattern (XRPD pattern) of the phosphate crystal form II is substantially as shown in FIG. 10.

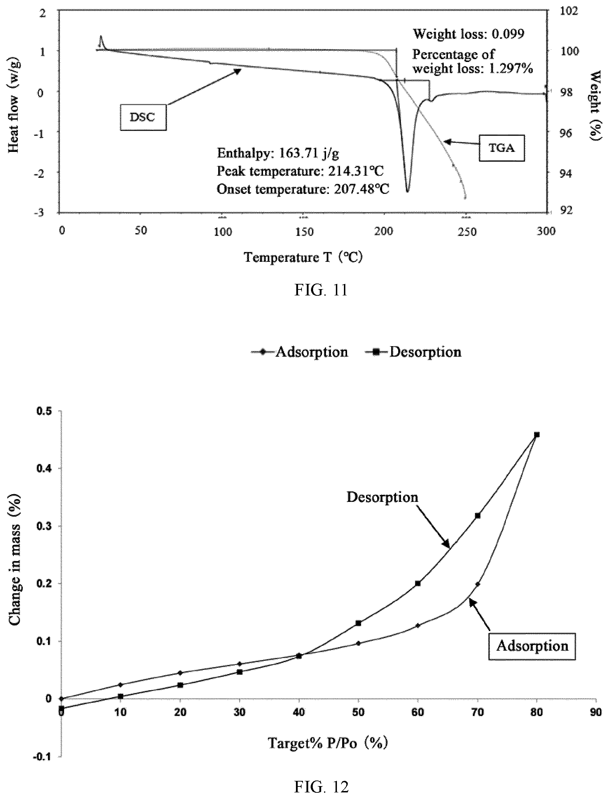

In some embodiments, a differential scanning calorimeter curve of the phosphate crystal form II has endothermic peaks at 207.48±3°C, 207.48±2°C, 207.48±1°C or 207.48±0.5°C. In some embodiments, the differential scanning calorimeter curve of the phosphate crystal form II has an onset temperature of 207.48°C±3°C, 207.48°C±2°C, 207.48°C±1°C or 207.48°C±0.5°C, and has a peak temperature of 214.31°C±3°C, 214.31°C±2°C, 214.31°C±1°C or 214.31°C±0.5°C. In some embodiments, the differential scanning calorimetry curve (DSC curve) of the phosphate crystal form II is substantially as shown in FIG. 11. In the embodiment as shown in FIG. 11, the phosphate salt form II of the compound of Formula (I) has a melting point of 207.48±0.5°C.

In some embodiments, a thermogravimetric analysis curve (TGA curve) of the phosphate crystal form II of the compound of Formula (I) is substantially as shown in FIG.11. In some embodiments, the TGA curve of the phosphate crystal form II of the compound of Formula (I) shows almost no weight loss near 100°C, and it begins to melt when heated to near the melting point of 207.48°C. In some embodiments, the TGA curve of the phosphate crystal form II of the compound of Formula (I) shows the phosphate crystal form II is an anhydrous substance.

In some embodiments, a dynamic vapor sorption spectrum (DVS spectrum) of the phosphate crystal form II of the compound of Formula (I) is substantially as shown in FIG. 12. In some embodiments, the DVS spectrum of the phosphate crystal form II of the compound of Formula (I) shows a moisture absorption weight gain of 0.45% at 80% relative humidity (RH).

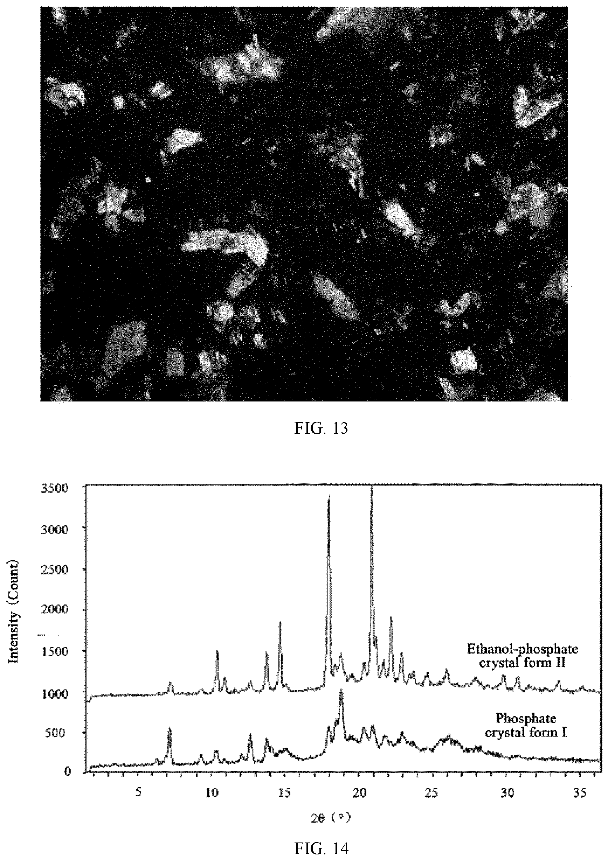

In some embodiments, a microscope image of the phosphate crystal form II of the compound of Formula (I) is substantially as shown in FIG. 13. In some embodiments, the microscope image of the phosphate crystal form II of the compound of Formula (I) shows the phosphate crystal form II is rod-shaped.

A second aspect of the present disclosure provides a preparation method of the L-tartrate of the compound of Formula (I).

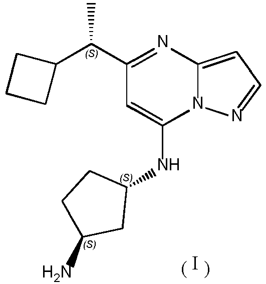

In some embodiments, a preparation method of the L-tartrate of the compound of Formula (I) comprises the following steps: subjecting the compound of Formula (I) to a salt-forming reaction with L-tartaric acid to form the L-tartrate of the compound of Formula (I).

In some embodiments, in the preparation method of the L-tartrate, the molar ratio of feed of L-tartaric acid to the compound of Formula (I) is (0.8-1.4):1. In some embodiments, the molar ratio of feed of L-tartaric acid to the compound of Formula (I) is (0.9-1.2):1.

In some embodiments, in the preparation method of the L-tartrate, the obtained L-tartrate of the compound of Formula (I) has the molar ratio of L-tartaric acid to the compound of Formula (I) of (0.8-1.2):1. In some embodiments, the obtained L-tartrate of the compound of Formula (I) has the molar ratio of L-tartaric acid to the compound of Formula (I) of (0.9-1.1):1. In some embodiments, the obtained L-tartrate of the compound of Formula (I) has the molar ratio of L-tartaric acid to the compound of Formula (I) of 1:1.

In some embodiments, a preparation method of a polymorph of the L-tartrate of the compound of Formula (I) is also provided, wherein the polymorph is the L-tartrate crystal form I, the method comprises the following steps:
subjecting the compound of Formula (I) to a salt-forming reaction with L-tartaric acid in an organic solvent to form a reaction solution; and
slowly cooling the reaction solution to obtain the L-tartrate crystal form I.

In some embodiments, the organic solvent is one or more selected from the group consisting of ethanol, acetonitrile, ethyl acetate, acetone and methanol.

In some embodiments, in the preparation method of the L-tartrate crystal form I, the molar ratio of feed of L-tartaric acid to the compound of Formula (I) is (0.8-1.4):1. In other embodiments, the molar ratio of feed of L-tartaric acid to the compound of Formula (I) is (0.9-1.2):1.

In some embodiments, in the preparation method of the L-tartrate crystal form I, the obtained L-tartrate crystal form I has the molar ratio of L-tartaric acid to the compound of Formula (I) of (0.8-1.2):1. In other embodiments, the obtained L-tartrate crystal form I has the molar ratio of L-tartaric acid to the compound of Formula (I) of (0.9-1.1):1. In an embodiment, the obtained L-tartrate crystal form I has the molar ratio of L-tartaric acid to the compound of Formula (I) of 1:1.

In some embodiments, a preparation method of the L-tartrate crystal form II is also provided, and the method comprises the following steps:
subjecting the compound of Formula (I) to a salt-forming reaction with L-tartaric acid in an organic solvent to form a reaction solution; and
slowly cooling the reaction solution, and adding an anti-solvent to obtain the L-tartrate crystal form II.

In some embodiments, the organic solvent is one or more selected from the group consisting of ethanol, acetonitrile, ethyl acetate, acetone and methanol.

In some embodiments, the anti-solvent is one or more selected from the group consisting of methyl tert-butyl ether, petroleum ether, n-heptane, n-hexane, cyclohexane, isopropanol, acetone, acetonitrile, and ethyl acetate, and the anti-solvent is different from the organic solvent. In other embodiments, the organic solvent is one or more selected from the group consisting of methyl tert-butyl ether, n-heptane, isopropanol and acetone.

In some embodiments, in the preparation method of the L-tartrate crystal form II, the molar ratio of feed of L-tartaric acid to the compound of Formula (I) is (0.8-1.4):1. In other embodiments, the molar ratio of feed of L-tartaric acid to the compound of Formula (I) is (0.9-1.2):1.

In some embodiments, in the preparation method of the L-tartrate crystal form II, the obtained L-tartrate crystal form II has the molar ratio of L-tartaric acid to the compound of Formula (I) of (0.8-1.2):1. In other embodiments, the obtained L-tartrate crystal form II has the molar ratio of L-tartaric acid to the compound of Formula (I) of (0.9-1.1):1. In an embodiment, the obtained L-tartrate crystal form II has the molar ratio of L-tartaric acid to the compound of Formula (I) of 1:1.

In some embodiments, a temperature of salt-forming reaction is -10°C to 90°C. For example, the temperature of salt-forming reaction can be any range (including the end value) consisted of the end values selected from the group consisting of -10°C, -5°C, 0°C, 5°C, 10°C, 15°C, 20°C, 25°C, 30°C, 35°C, 40°C, 45°C, 50°C, 55°C, 60°C, 65°C, 70°C, 75°C, 80°C, 85°C and 90°C, and is not specifically limited.

In some embodiments, the reaction solution is slowly cooled to -10°C to 60°C. In some embodiments, the reaction solution is slowly cooled to 0°C to room temperature. In some embodiments, the reaction solution is slowly cooled to room temperature.

In some embodiments, the molar concentration of L-tartaric acid is 0.1 mol/L to 5 mol/L. For example, the molar concentration of L-tartaric acid can be 0.1 mol/L, 1mol/L, 1.5 mol/L, 2 mol/L, 2.5 mol/L, 3 mol/L, 3.5 mol/L, 4 mol/L, 4.5 mol/L, or 5 mol/L. In other embodiments, the molar concentration of L-tartaric acid is 0.5 mol/L to 3 mol/L. In other embodiments, the molar concentration of L-tartaric acid is 1 mol/L to 2 mol/L.

In some embodiments, the preparation method of the L-tartrate crystal form I comprises the following steps:
(BI-a) dissolving the compound of Formula (I) in a solvent, adding an aqueous solution of L-tartaric acid, and stirring to react; and
(BI-b) cooling the reaction solution obtained in step (BI-a), precipitating a solid, performing solid-liquid separation, and collecting the solid phase to obtain the L-tartrate crystal form I of compound of Formula (I).

In some embodiments, a temperature of the reaction in step (BI-a) is 10°C to 60°C.

In some embodiments, a molar ratio of the compound of Formula (I) to L-tartaric acid in the aqueous solution of L-tartaric acid in step (BI-a) is 1:(0.8-1.4). In some embodiments, a molar ratio of the compound of Formula (I) to L-tartaric acid in the aqueous solution of L-tartaric acid in step (BI-a) is 1:(0.9-1.2).

In some embodiments, the molar concentration of the aqueous solution of L-tartaric acid in step (BI-a) is 0.1 mol/L to 5 mol/L. In other embodiments, the molar concentration of the aqueous solution of L-tartaric acid in step (BI-a) is 0.5 mol/L to 3 mol/L. In other embodiments, the molar concentration of the aqueous solution of L-tartaric acid in step (BI-a) is 1 mol/L to 2 mol/L.

In some embodiments, the solvent in step (BI-a) is ethanol.

n some embodiments, the stirring in step (BI-a) is performed at the temperature of 30°C to 60°C, and then at room temperature. In some embodiments, the stirring in step (BI-a) is performed at the temperature of 30°C to 60°C for 2-5 h, and then at room temperature. In some embodiments, the stirring in step (BI-a) is performed at the temperature of 30°C to 60°C for 2-5 h, and then at room temperature for 7-16 h.

In some embodiments, in step (BI-b), the reaction solution is cooled to -10°C to 10°C. In some embodiments, the reaction solution is cooled to -5°C to 5°C. In some embodiments, the reaction solution is cooled to about 0°C.

In some embodiments, a time of cooling the reaction solution in step (BI-b) is 0.25-3 h. In some embodiments, the time of cooling the reaction solution is 0.5-2 h. In some embodiments, the time of cooling the reaction solution is 0.5-1 h.

In some embodiments, the separation method in step (BI-b) is selected from the group consisting of centrifugal separation and filtration separation.

In some embodiments, after the separation in step (BI-b), the method further comprises the step of evaporating the solvent or drying.

In some embodiments, the drying step after the separation in step (BI-b) is performed at the temperature of 25°C to 70°C.

In some embodiments, the preparation method of the L-tartrate crystal form II comprises the following steps:
(BII-a) dissolving the compound of Formula (I) in a solvent, adding an aqueous solution of L-tartaric acid, and stirring to react; and
(BII-b) cooling the reaction solution obtained in step (BII-a), adding an anti-solvent for crystallization, separating solid and liquid, and collecting the solid phase to obtain L-tartrate crystal form II of the compound of Formula (I).

In some embodiments, a temperature of the reaction in step (BII-a) is 10°C to 60°C.

In some embodiments, a molar ratio of the compound of Formula (I) to the L-tartaric acid in the aqueous solution of L-tartaric acid in step (BII-a) is 1:(0.8-1.4). In other embodiments, the molar ratio of the compound of Formula (I) to the L-tartaric acid in the aqueous solution of L-tartaric acid is 1:(0.9-1.2).

In some embodiments, the molar concentration of the aqueous solution of L-tartaric acid in step (BII-a) is 0.1 mol/L to 5 mol/L. In some embodiments, the molar concentration of the aqueous solution of L-tartaric acid is 0.5 mol/L to 3 mol/L. In other embodiments, the molar concentration of the aqueous solution of L-tartaric acid is 1 mol/L to 2 mol/L.

In some embodiments, the solvent in step (BII-a) is methanol.

n some embodiments, the stirring in step (BII-a) is performed at the temperature of 30°C to 60°C, and then at room temperature. In some embodiments, the stirring in step (BII-a) is performed at the temperature of 30°C to 60°C for 2-5 h, and then at room temperature. In some embodiments, the stirring in step (BII-a) is performed at the temperature of 30°C to 60°C for 2-5 h, and then at room temperature for 7-16 h.

In some embodiments, in step (BII-b), the reaction solution is cooled to -10°C to 10°C. In some embodiments, the reaction solution is cooled to -5°C to 5°C. In some embodiments, the reaction solution is cooled to about 0°C.

In some embodiments, a time of cooling the reaction solution in step (BII-b) is 0.25-3 h. In some embodiments, the time of cooling the reaction solution is 0.5-2 h. In some embodiments, the time of cooling the reaction solution is 0.5-1 h.

In some embodiments, the anti-solvent in step (BII-b) is one or more selected from the group consisting of methyl tert-butyl ether, n-heptane, n-hexane, cyclohexane, acetone, acetonitrile, and ethyl acetate, and the solvent is different from the anti-solvent. In some embodiments, the anti-solvent is one or more selected from the group consisting of methyl tert-butyl ether, acetone, acetonitrile and ethyl acetate. In some embodiments, the anti-solvent is methyl tert-butyl ether.

In some embodiments, the separation method in step (BII-b) is selected from the group consisting of centrifugal separation and filtration separation.

In some embodiments, after the separation in step (BII-b), the method further comprises the step of evaporating the solvent or drying.

In some embodiments, the drying step after the separation in step (BII-b) is performed at the temperature of 25°C to 70°C.

In some embodiments, the preparation method of the amorphous L-tartrate comprises the following steps:
(C-a) dissolving the compound of Formula (I) in a solvent, adding an aqueous solution of L-tartaric acid, and stirring to react; and
(C-b) cooling the reaction solution obtained in step (C-a), precipitating a solid, performing solid-liquid separation, and collecting the solid phase to obtain the amorphous L-tartrate.

In some embodiments, a temperature of the reaction in step (C-a) is 10°C to 60°C.

In some embodiments, a molar ratio of the compound of Formula (I) to the L-tartaric acid in the aqueous solution of L-tartaric acid in step (C-a) is 1:(0.8-1.4). In some embodiments, a molar ratio of the compound of Formula (I) to the L-tartaric acid in the aqueous solution of L-tartaric acid in step (C-a) is 1:(0.9-1.2).

In some embodiments, the molar concentration of the aqueous solution of L-tartaric acid in step (C-a) is 0.1 mol/L to 5 mol/L. In some embodiments, the molar concentration of the aqueous solution of L-tartaric acid is 0.5 mol/L to 3 mol/L. In some embodiments, the molar concentration of the aqueous solution of L-tartaric acid is 1 mol/L to 2 mol/L.

In some embodiments, the solvent in step (C-a) is acetone.

In some embodiments, the stirring in step (C-a) is performed at the temperature of 30°C to 60°C, and then at room temperature. In some embodiments, the stirring in step (C-a) is performed at the temperature of 30°C to 60°C for 2-5 h, and then at room temperature. In some embodiments, the stirring in step (C-a) is performed at the temperature of 30°C to 60°C for 2-5 h, and then at room temperature for 7-16 h.

In some embodiments, in step (C-b), the reaction solution is cooled to -10°C to 10°C. In some embodiments, the reaction solution is cooled to -5°C to 5°C. In some embodiments, the reaction solution is cooled to about 0°C.

In some embodiments, a time of cooling the reaction solution in step (C-b) is 0.25-3 h. In some embodiments, the time of cooling the reaction solution is 0.5-2 h. In some embodiments, the time of cooling the reaction solution is 0.5-1 h.

In some embodiments, the separation method in step (C-b) is selected from the group consisting of centrifugal separation and filtration separation.

In some embodiments, after the separation in step (C-b), the method further comprises the step of evaporating the solvent or drying.

In some embodiments, the drying step after the separation in step (C-b) is performed at the temperature of 25°C to 70°C.

A third aspect of the present disclosure provides a preparation method of a phosphate of the compound of Formula (I).

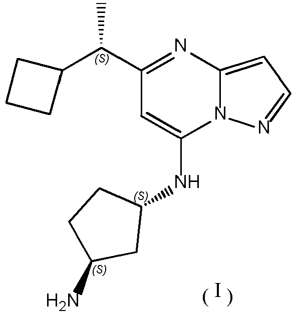

The preparation method of the phosphate of the compound of Formula (I) comprises the following steps: subjecting the compound of Formula (I) to a salt-forming reaction with phosphoric acid in the presence of an organic solvent to form the phosphate of the compound of Formula (I).

In some embodiments, the phosphate is a second type of phosphate of the compound of Formula (I). In some embodiments, the molar ratio of feed of the compound of Formula (I) to phosphoric acid is 1:(0.8-1.2). For example, the molar ratio of feed of the compound of Formula (I) to phosphoric acid can be 1:0.8, 1:0.9, 1:1.0, 1:1.1 or 1:1.2.

In some embodiments, the molar ratio of feed of the compound of Formula (I) to phosphoric acid is 1:(0.9-1.1). In some embodiments, the molar ratio of feed of the compound of Formula (I) to phosphoric acid is 1:1. In some embodiments, the molar ratio of feed of the compound of Formula (I) to phosphoric acid is 1:0.95.

In some embodiments, the molar ratio of the compound of Formula (I) to phosphoric acid in the phosphate is 1:(0.8-1.2). In some embodiments, the molar ratio of the compound of Formula (I) to phosphoric acid in the phosphate is 1:(0.9-1.1). In some embodiments, the molar ratio of the compound of Formula (I) to phosphoric acid in the phosphate can be 1:0.8, 1:0.9, 1:1, 1:1.1 or 1:1.2. In some embodiments, the molar ratio of the compound of Formula (I) to phosphoric acid in the phosphate is 1:1.

In some embodiments, the organic solvent is one or more selected from the group consisting of ethanol, ethyl acetate, acetonitrile and acetone. In some embodiments, the organic solvent is ethanol, acetone, or mixture thereof. In some embodiments, the organic solvent is ethanol.

In some embodiments, the organic solvent is one or more of methanol, ethyl acetate and acetone. In some embodiments, the organic solvent is one or more of methanol and ethyl acetate. In some embodiments, the organic solvent is a mixture of methanol and ethyl acetate.

In some embodiments, the phosphate of the compound of Formula (I) obtained has the molar ratio of the compound of Formula (I) to phosphoric acid of 1:1.

In some embodiments, a preparation method of the phosphate crystal form II is also provided, and the method comprises the following steps:
(i) subjecting the compound of Formula (I) to a salt-forming reaction with phosphoric acid in ethanol, methanol and/or acetone to precipitate a solid; and
(ii) collecting the solid to obtain the phosphate crystal form II;
wherein, the molar ratio of feed of the compound of Formula (I) to phosphoric acid is 1:(0.8-1.2), for example, it can be 1:0.8, 1:0.9, 1:1.0, 1:1.1 or 1:1.2. In some embodiments, the molar ratio of feed of the compound of Formula (I) to phosphoric acid is 1:(0.9-1.1). In an embodiment, the molar ratio of feed of the compound of Formula (I) to phosphoric acid is 1:0.95. In another embodiment, the molar ratio of feed of the compound of Formula (I) to phosphoric acid is 1:1.

In some embodiments, the salt-forming reaction is performed in one or both of ethanol and acetone.

In some embodiments, the salt-forming reaction is performed in ethanol. In some embodiments, the salt-forming reaction is performed in methanol.

In some embodiments, the preparation method of the phosphate crystal form II comprises the following steps:
(i-1) subjecting the ethanol solution of the compound of Formula (I) to a salt-forming reaction with an ethanol solution of phosphoric acid to precipitate a solid; and
(ii-1) collecting the solid to obtain the phosphate crystal form II;
wherein, the molar ratio of feed of the compound of Formula (I) to phosphoric acid is 1:(0.8-1.2), for example, it can be 1:0.8, 1:0.9, 1:1.0, 1:1.1 or 1:1.2. In some embodiments, the molar ratio of feed of the compound of Formula (I) to phosphoric acid is 1:(0.9-1.1). In an embodiment, the molar ratio of feed of the compound of Formula (I) to phosphoric acid is 1:1.

In some embodiments, the preparation method of the phosphate crystal form II comprises the following steps:
(i-1) subjecting the ethanol solution of the compound of Formula (I) to a salt-forming reaction with the ethanol solution of phosphoric acid at reflux temperature, cooling the temperature to -10°C to 60°C to precipitate a solid; and
(ii-1) collecting the solid to obtain the phosphate crystal form II;
wherein, the molar ratio of feed of the compound of Formula (I) to phosphoric acid is 1:(0.8-1.2), for example, it can be 1:0.8, 1:0.9, 1:1.0, 1:1.1 or 1:1.2. In some embodiments, the molar ratio of feed of the compound of Formula (I) to phosphoric acid is 1:(0.9-1.1). In an embodiment, the molar ratio of feed of the compound of Formula (I) to phosphoric acid is 1:1.

In some embodiments, the phosphate crystal form II has the molar ratio of the compound of Formula (I) to phosphoric acid of 1:1.

For example, the temperature of salt-forming reaction is -10°C to 90°C, for example, it can be any range (including the end value) consisted of the end values selected from the group consisting of -10°C, -5°C, 0°C, 5°C, 10°C, 15°C, 20°C, 25°C, 30°C, 35°C, 40°C, 45°C, 50°C, 55°C, 60°C, 65°C, 70°C, 75°C, 80°C, 85°C and 90°C, and is not specifically limited.

In some embodiments, the method for collecting the solid in step (ii) or (ii-1) can be centrifugal separation and filtration separation.

In some embodiments, after collecting the solid in step (ii) or (ii-1), the method further comprises the step of evaporating the solvent or drying.

In some embodiments, the drying step is performed at the temperature of 25°C to 70°C.

In some embodiments, the phosphate is a first type of phosphate of the compound of Formula (I). The molar ratio of feed of the compound of Formula (I) to phosphoric acid is 1:(1.8-2.6), for example, it can be 1:1.8, 1:1.9, 1:2.0, 1:2.1, 1:2.2, 1:2.3, 1:2.4, 1:2.5 or 1:2.6; preferably 1:(1.9-2.3), and more preferably 1:(2.0-2.2).

In some embodiments, the phosphate has the molar ratio of phosphoric acid to the compound of Formula (I) is (1.8-2.4):1. In some embodiments, the molar ratio of phosphoric acid to the compound of Formula (I) can be 1.8:1, 1.9:1, 2.0:1, 2.1:1, 2.2:1, 2.3:1 or 2.4:1. In some embodiments, the molar ratio of phosphoric acid to the compound of Formula (I) is (1.9-2.3):1. In some embodiments, the molar ratio of phosphoric acid to the compound of Formula (I) is 2:1.

In some embodiments, the organic solvent is one or more selected from the group consisting of ethanol, ethyl acetate, acetone and methanol.

In some embodiments, the phosphate of the compound of Formula (I) obtained has the molar ratio of phosphoric acid to the compound of Formula (I) of 2:1.

In some embodiments, the preparation method of the phosphate crystal form I comprises the following steps:
(AI-a) dissolving the compound of Formula (I) in an organic solvent, adding an aqueous solution of phosphoric acid, and stirring to react; and
(AI-b) cooling the reaction solution obtained in step (AI-a), adding an anti-solvent for crystallization, performing solid-liquid separation, and collecting the solid phase to obtain the phosphate crystal form I of the compound of Formula (I).

In an embodiment, a temperature of the reaction in step (AI-a) is 10°C to 60°C.

In an embodiment, the molar ratio of the compound of Formula (I) to phosphoric acid in the aqueous solution of phosphoric acid is 1:(1.8-2.6), for example, it can be 1:1.8, 1:1.9, 1:2.0, 1:2.1, 1:2.2, 1:2.3, 1:2.4, 1:2.5 or 1:2.6; preferably 1:(1.9-2.3), and more preferably 1:(2.0-2.2).

In some embodiments, the molar concentration of the aqueous solution of phosphoric acid is 0.1 mol/L to 5 mol/L, preferably 0.5 mol/L to 3 mol/L, and more preferably 1 mol/L to 2 mol/L.

In some embodiments, in step (AI-b), the reaction solution is cooled to -10°C to 10°C, and preferably -5°C to 5°C; in some of these embodiments, the reaction solution is cooled to about 0°C.

In some embodiments, a time of cooling the reaction solution in step (AI-b) is 0.5-2 h; in some of these embodiments, the time of cooling the reaction solution is 0.5 h.

In an embodiment, the anti-solvent is one or more selected from the group consisting of methyl tert-butyl ether, petroleum ether, n-heptane, n-hexane, cyclohexane, isopropanol, acetone, acetonitrile, and ethyl acetate, and the anti-solvent is different from the organic solvent; preferably, the anti-solvent is one or more selected from the group consisting of methyl tert-butyl ether, n-heptane, isopropanol and acetone.

In an embodiment, the separation method in step (AI-b) is selected from the group consisting of centrifugal separation and filtration separation.

In an embodiment, after the separation in step (AI-b), the method further comprises the step of evaporating the solvent or drying.

In some embodiments, the drying step after the separation in step (AI-b) is performed at the temperature of 25°C to 70°C, for example, the temperature can be 25°C, 30°C, 40°C, 50°C, 60°C or 70°C, etc, and is not specifically limited.

In an embodiment, the temperature of the reaction in step (AI-a) is 10°C to 60°C; and/or the molar ratio of the compound of Formula (I) to phosphoric acid in the aqueous solution of phosphoric acid is 1:(1.8-2.4), preferably 1:(1.9-2.3), and more preferably 1:(2.0-2.2); and/or the molar concentration of the aqueous solution of phosphoric acid is 0.1 mol/L to 5 mol/L, preferably 0.5 mol/L to 3 mol/L, and more preferably 1 mol/L to 2 mol/L; and/or in step (AI-b), the reaction solution is cooled to -10°C to 10°C, preferably -5°C to 5°C, and more preferably about 0°C; and/or the cooling time of the reaction solution in step (AI-b) is 0.5 to 2 hours, more preferably 0.5 hours; and/or the anti-solvent is one or more selected from the group consisting of methyl tert-butyl ether, petroleum ether, n-heptane, n-hexane, cyclohexane, isopropanol, acetone, acetonitrile, and ethyl acetate, and the anti-solvent is different from the organic solvent; preferably, the anti-solvent is one or more selected from the group consisting of methyl tert-butyl ether, n-heptane, isopropanol and acetone; and/or the separation method in step (AI-b) is selected from the group consisting of centrifugal separation and filtration separation; and/or after the separation in step (AI-b), the method further comprises the step of evaporating the solvent or drying; and/or the drying step after the separation in step (AI-b) is performed at the temperature of 25°C to 70°C.

A fourth aspect of the present disclosure provides a pharmaceutical composition, the pharmaceutical composition comprises:
(a) the L-tartrate or phosphate of the compound of Formula (I) of the first aspect of the present disclosure; and (b) a pharmaceutically acceptable carrier.

In some embodiments, the pharmaceutical composition comprises (a) the L-tartrate of the compound of Formula (I), the first type of phosphate of the compound of Formula (I), or the second type of phosphate of the compound of Formula (I) of the first aspect of the present disclosure; and (b) a pharmaceutically acceptable carrier.

In some embodiments, the pharmaceutical composition comprises (a) the L-tartrate crystal form I of the compound of Formula (I), the L-tartrate crystal form II of the compound of Formula (I), the amorphous L-tartrate of the compound of Formula (I), the phosphate crystal form I of the compound of Formula (I) or the phosphate crystal form II of the compound of Formula (I) of the first aspect of the present disclosure; and (b) a pharmaceutically acceptable carrier.

A fifth aspect of the present disclosure provides use of the pharmaceutically acceptable salt of the compound of Formula (I) described in the first aspect of the present disclosure or the pharmaceutical composition described in the fourth aspect of the present disclosure in the manufacture of a kinase inhibitor. In particular, the pharmaceutically acceptable salt of the compound of Formula (I) comprises the L-tartrate of the compound of Formula (I), the first type of phosphate of the compound of Formula (I), or the second type of phosphate of the compound of Formula (I). In some embodiments, the pharmaceutically acceptable salt of the compound of Formula (I) comprises the L-tartrate crystal form I of the compound of Formula (I), the L-tartrate crystal form II of the compound of Formula (I), the amorphous L-tartrate of the compound of Formula (I), the phosphate crystal form I of the compound of Formula (I) or the phosphate crystal form II of the compound of Formula (I). In some embodiments, the kinase inhibitor is a CDK9 inhibitor.

A sixth aspect of the present disclosure provides use of the pharmaceutically acceptable salt of the compound of Formula (I) described in the first aspect of the present disclosure or a pharmaceutical composition described in the fourth aspect of the present disclosure in the manufacture of a medicament for treating and/or preventing a disease associated with or mediated by CDK9 activity. In particular, the pharmaceutically acceptable salt of the compound of Formula (I) comprises the L-tartrate of the compound of Formula (I), the first type of phosphate of the compound of Formula (I), or the second type of phosphate of the compound of Formula (I). In some embodiments, the pharmaceutically acceptable salt of the compound of Formula (I) comprises the L-tartrate crystal form I of the compound of Formula (I), the L-tartrate crystal form II of the compound of Formula (I), the amorphous L-tartrate of the compound of Formula (I), the phosphate crystal form I of the compound of Formula (I) or the phosphate crystal form II of the compound of Formula (I).

The present disclosure further provides the pharmaceutically acceptable salt of the compound of Formula (I) described in the first aspect of the present disclosure or the pharmaceutical composition described in the fourth aspect of the present disclosure, for treating and/or preventing a disease associated with or mediated by CDK9 activity.

The present disclosure further provides a method for inhibiting CDK9 activity, comprising administering to a subject in need thereof a therapeutically effective amount of the pharmaceutically acceptable salt of the compound of Formula (I) described in the first aspect of the present disclosure or the pharmaceutical composition described in the fourth aspect of the present disclosure.

The present disclosure further provides a method for treating a disease associated with or mediated by CDK9 activity, comprising administering to a subject in need thereof a therapeutically effective amount of the pharmaceutically acceptable salt of the compound of Formula (I) described in the first aspect of the present disclosure or the pharmaceutical composition described in the fourth aspect of the present disclosure.

In some embodiments, the disease comprises a hyperproliferative disease, a virus-induced infectious disease, and a cardiovascular disease.

In some embodiments, the disease is a hyperproliferative disease. In some embodiments, the hyperproliferative disease comprises angiogenic or angioproliferative disorders, mesangial cell proliferative diseases, and solid tumors. The solid tumors are, for example, cancers of the breast, respiratory tract, brain, reproductive organs, digestive tract, urinary tract, eye, liver, skin, head and neck, thyroid or parathyroid glands, and distant metastases thereof, etc. In some embodiments, the disease is one or more selected from the group consisting of lymphoma, sarcoma, and leukemia. In some embodiments, the disease is a cancer. The cancer is, for example, pancreatic cancer, breast cancer, ovarian cancer, cervical cancer, or leukemia, etc. In some embodiments, the disease comprises solid tumors and hematological tumors.

In the present disclosure, the disease associated with or mediated by CDK9 activity comprise a disease associated with or involving CDK9 activity (eg, overactivity of CDK9), and conditions that accompany these diseases. Overactivity of CDK9 refers to increased CDK9 enzyme activity compared to normal, non-diseased cells, or to increased CDK9 activity resulting in undesired cell proliferation, or reduced or insufficient programmed cell death (apoptosis), or to mutations resulting in constitutive activation of CDK9.

Hyperproliferative disease comprises a disease involving the undesired or uncontrolled proliferation of cells, and comprises a disease involving reduced or insufficient programmed cell death (apoptosis). The pharmaceutically acceptable salt of the compound of Formula (I) of the present disclosure, or the pharmaceutical composition comprising the pharmaceutically acceptable salt of the compound of Formula (I) can be used to prevent, inhibit, block, reduce, lower, control, etc., cell proliferation and/or cell division, and/or induce cell apoptosis. The treatment and/or prevention of the disease associated with CDK9 activity or mediated by CDK9 activity comprises administering to a subject in need thereof (including a mammal, such as a human) a pharmaceutically acceptable salt of a compound of Formula (I) of the present disclosure, or a pharmaceutical composition comprising a pharmaceutically acceptable salt of a compound of Formula (I) effective for treating or preventing the disease.

In the present disclosure, the phosphate and L-tartrate of the compound of Formula (I) have higher solubility than the free base, and the L-tartrate crystal form I of the compound of Formula (I), the L-tartrate crystal form II of the compound of Formula (I), the amorphous L-tartrate of the compound of Formula (I), the phosphate crystal form I of the compound of Formula (I) or the phosphate crystal form II of the compound of Formula (I) all have good physical stability and are suitable for drug development. In particular, the phosphate crystal form I of the compound of Formula (I) can be transformed into the more stable phosphate crystal form II under appropriate conditions, such as in ethanol. Compared with the phosphate crystal form I of the compound of Formula (I), the phosphate crystal form II of the compound of Formula (I) has lower hygroscopicity and better crystallinity and stability.

### DETAILED DESCRIPTION OF THE DRAWINGS

In order to more clearly illustrate the embodiments of the present disclosure or the technical solutions in the related technologies, the drawings required for use in the embodiments or the related technical descriptions will be briefly introduced below. Obviously, the drawings described below are only embodiments of the present application. For those skilled in the art, other drawings can be obtained based on the disclosed drawings without creative effort.
FIG. 1 shows an X-ray powder diffraction (XRPD) pattern of a phosphate crystal form I of the compound of Formula (I).
FIG. 2 shows a differential scanning calorimetry (DSC) spectrum and thermogravimetric analysis (TGA) spectrum of a phosphate crystal form I of the compound of Formula (I).
FIG. 3 shows a dynamic vapor sorption (DVS) curve of the phosphate crystal form I of the compound of Formula (I).
FIG. 4 shows a microscope image of a phosphate crystal form I of the compound of Formula (I).
FIG. 5 shows an X-ray powder diffraction (XRPD) pattern of an L-tartrate crystal form I of the compound of Formula (I).
FIG. 6 shows a thermogravimetric analysis (TGA) spectrum of an L-tartrate crystal form I of the compound of Formula (I).
FIG. 7 shows an X-ray powder diffraction (XRPD) pattern of an L-tartrate crystal form II of the compound of Formula (I).
FIG. 8 shows an X-ray powder diffraction (XRPD) pattern of an amorphous L-tartrate of the compound of Formula (I).
FIG. 9 shows an ellipsoid graph of the molecular stereostructure of a single crystal of the compound of Formula (II).
FIG. 10 shows an X-ray powder diffraction (XRPD) pattern of a phosphate crystal form II of the compound of Formula (I).
FIG. 11 shows a differential scanning calorimetry (DSC) spectrum and thermogravimetric analysis (TGA) spectrum of a phosphate crystal form II of the compound of Formula (I).
FIG. 12 shows a dynamic vapor sorption (DVS) curve of a phosphate crystal form II of the compound of Formula (I).
FIG. 13 shows a microscope image of a phosphate crystal form II of the compound of Formula (I).
FIG. 14 shows a comparative X-ray powder diffraction (XRPD) pattern of the conversion of the phosphate crystal form I of the compound of Formula (I) into the phosphate crystal form II of the compound of Formula (I) in ethanol.
FIG. 15 show a graph of XRPD change of the L-tartrate salt form I of the compound of Formula (I) under high temperature (60°C) and accelerated (40°C-75% RH) conditions.
FIG. 16 shows a graph of XRPD change of the phosphate crystal form I of the compound of Formula (I) under a high temperature condition.
FIG. 17 shows a graph of tumor volume changes following once-daily oral administration of Compound D and a compound of Formula (I).
FIG. 18 shows a graph of body weight changes in mice following once-daily oral administration of Compound D and a compound of Formula (I).

### DETAILED DESCRIPTION

In order to make the above-mentioned purposes, features and advantages of the present disclosure more obvious and understandable, the detailed description of the present disclosure are described in detail below. In the following description, many specific details are set forth to facilitate a full understanding of the present disclosure. However, the present disclosure can be implemented in many other ways different from those described herein, and those skilled in the art can make similar improvements without violating the connotation of the present disclosure, so the present disclosure is not limited by the specific embodiments disclosed below.

The "the crystalline of the present disclosure", "the crystal form of the present disclosure", "the polymorph of the present disclosure" and the like used herein may be used interchangeably.

In the present disclosure, the compound of Formula (I) is (1S,3S)-N1-(5-((S)-1-cyclobutylethyl)pyrazolo[1,5-a]pyrimidin-7-yl)cyclopentan-1,3-diamine, having the structure shown as follows:

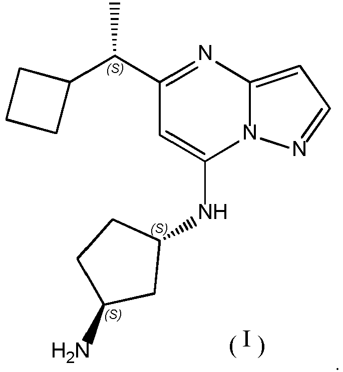

The present disclosure further comprises the phosphate and L-tartrate of the compound of Formula (I).

Polymorph: solids exist in either amorphous form or crystalline form. In the case of crystalline form, the molecules are positioned in a three-dimensional lattice site. When a compound crystallizes from a solution or slurry, it can crystallize in different spatial arrangements (a property known as "polymorphism"), forming crystals with different crystalline forms, which are known as "polymorphs". Different polymorphs of a given substance may differ from one another in one or more physical properties, such as solubility and dissolution rate, true specific gravity, crystal shape, packing spectrum, flowability and/or solid state stability.

Crystallization: crystallization on a production scale can be accomplished by manipulating the solution so that the solubility limit of the compound of interest is exceeded. This can be accomplished in a variety of ways, for example, by dissolving the compound at a relatively high temperature and then cooling the solution to below the saturation limit. Alternatively, the volume of the liquid can be reduced by boiling, atmospheric evaporation, vacuum drying, or by some other method. The solubility of the compound of interest can be reduced by adding an anti-solvent or a solvent or a mixture of such solvents in which the compound has a low solubility. Another alternative is to adjust the pH to reduce solubility. For a detailed description of crystallization, see Crystallization, 3rd edition, JW Mullens, Butterworth-Heineman Ltd., 1993, ISBN 0750611294.

The "suspension shaking" mentioned in the present disclosure refers to a method of mixing the compound of Formula (I) and the corresponding acid or the solution of the corresponding acid in a suitable solvent to form a turbid solution and then shaking to obtain a crystal. The suitable solvent may be water or an organic solvent.

The "suspension centrifugation" mentioned in the present disclosure refers to a method of mixing the compound of Formula (I) and the corresponding acid or the solution of the corresponding acid in a suitable solvent to form a turbid solution and then centrifuging to obtain a crystal. The suitable solvent may be water or an organic solvent.

The "slow volatilization" described in the present disclosure refers to a method in which a solution containing the compound of Formula (I) and a corresponding acid is placed at a certain temperature to slowly volatilize the solvent to obtain a crystal.

The "adding an anti-solvent" or "addition of an anti-solvent" described in the present disclosure refers to a method of adding another suitable solvent to a solution of the compound of Formula (I) and then precipitating the resulting crystal.

If it is desired that salt formation and crystallization occur simultaneously, the addition of an appropriate acid or base may result in direct crystallization of the desired salt if the salt is less soluble in the reaction medium than the starting materials. Likewise, completion of the synthesis reaction in a medium in which the final desired form is less soluble than the reactants may result in direct crystallization of the final product.

Optimization of crystallization can include seeding the crystallization medium with crystals of the desired form. In addition, many crystallization methods use a combination of the above strategies. One embodiment dissolves the compound of interest in a solvent at elevated temperature, followed by controlled addition of an appropriate volume of anti-solvent to bring the system just below saturation level. At this point, a seed crystal in the desired form may be added (while maintaining the integrity of the seed crystal) and the system cooled to complete crystallization. As used herein, the term "about" refers to within ±5 on the basis of a given value.

Herein, when referring to the units of a data range, if there is a unit only after the right endpoint, it means that the units of the left and right endpoints are the same. For example, 3-5 h means that the units of the left endpoint "3" and the right endpoint "5" are both h (hours).

Herein, "preferably" and "better" are only used to describe implementation methods or examples with better effects. It should be understood that they do not constitute a limitation on the scope of protection of the present disclosure. If there are multiple "preferably" appear in a technical solution, unless otherwise specified and there is no contradiction or mutual restriction, each "preferably" shall be independent of the other.

The terms "or/and" and "and/or" used herein include any one of two or more related listed items, and also include any and all combinations of the related listed items, wherein the any and all combinations include any combination of two related listed items, any more related listed items, or all related listed items. It should be noted that when at least three items are connected by at least two conjunctions selected from "and/or" and "or/and", it should be understood that in the present application, the technical solution undoubtedly includes technical solutions connected by "logical and", and also undoubtedly includes technical solutions connected by "logical or".

As used herein, "plurality", "multiple", "multiple times", etc., unless otherwise specified, refer to a quantity greater than or equal to 2. For example, "multiple" means greater than or equal to two.

Herein, the technical features described in an open manner include closed technical solutions composed of the listed features, and also include open technical solutions containing the listed features.

As used herein, the term "polymorph of the present disclosure" includes, but is not limited to, the phosphate form I of the compound of Formula (I), the phosphate form II of the compound of Formula (I), the L-tartrate form I of the compound of Formula (I), and the L-tartrate form II of the compound of Formula (I).

The present disclosure also includes a phosphate of the compound of Formula (I), in particular a monophosphate, and a crystalline form II of the phosphate of the compound of Formula (I).

The "compound of Formula (I)", "free base of the compound of Formula (I)" and "free base" are used interchangeably.

The "polymorph of the compound of Formula (I)" and "polymorph of the free base of the compound of Formula (I)" are used interchangeably.

Herein, certain crystal forms can be converted into each other, and thus the present disclosure also provides a method for converting some crystal forms into each other.

### Pharmaceutical composition and use thereof

Generally, the pharmaceutically acceptable salt of the compound of Formula (I) disclosed herein as an active ingredient can be administered in a suitable dosage form together with one or more pharmaceutical carriers.

Specifically, the phosphate, the L-tartrat and the polymorph thereof of the compound of Formula (I) disclosed herein as an active ingredient can be administered in a suitable dosage form together with one or more pharmaceutical carriers.

"Pharmaceutically acceptable carrier" refers to a non-toxic, inert, solid, semi-solid substance or liquid filler, diluent, encapsulating material or auxiliary formulation or any type of excipient, which is compatible with the subject to be administered (in some embodiments, a mammal, in one embodiment, a human), and is suitable for transporting the active substance of the present disclosure to the target site without terminating its activity.

The pharmaceutical composition disclosed herein are formulated, dosed and administered in a manner consistent with medical practice. The "therapeutically effective amount" of the active ingredient administered is determined by factors such as the specific condition to be treated, the individual being treated, the cause of the condition, the target of the drug, and the mode of administration.

The present disclosure provides the pharmaceutically acceptable salt of the compound of Formula (I) described in the first aspect of the present disclosure, the pharmaceutical composition described in the fourth aspect of the present disclosure, which can be used as a medicine for treating and/or preventing a disease associated with or mediated by CDK9 activity.

The present disclosure provides a method for inhibiting CDK9 activity, comprising administering to a subject a therapeutically effective amount of the pharmaceutically acceptable salt of the compound of Formula (I) described in the first aspect of the present disclosure or the pharmaceutical composition described in the fourth aspect of the present disclosure.

As used herein, "therapeutically effective amount" refers to an amount of a pharmaceutically acceptable salt of the compound of Formula (I) of the present disclosure that will induce a biological or medical response in a subject, such as reducing or inhibiting enzyme or protein activity or improving symptoms, alleviating symptoms, relieving or delaying disease progression, or preventing disease, etc.

As used herein, "subject" refers to an animal, preferably a mammal, more preferably a human. The term "mammal" refers to a warm-blooded vertebrate mammal including a cat, dog, rabbit, bear, fox, wolf, monkey, deer, mouse, pig and human.

"Treatment" means to lessen, slow the progression, attenuate, prevent, or maintain an existing disease or condition (e.g., cancer). Treatment also includes curing, preventing the development of, or alleviating to some extent, one or more symptoms of a disease or condition.

### EXAMPLES

The present disclosure will be further illustrated in conjunction with the following specific examples. It should be understood that the examples are only used to illustrate the present disclosure and not to limit the scope of the present disclosure. For the experimental procedures without specific conditions indicated in the following examples are generally performed according to conventional conditions or as recommended by the manufacturer. Percentages and parts are by weight unless otherwise indicated. The terms used herein have the same meanings as those skilled in the art unless otherwise defined. In addition, any methods and materials similar or equivalent to those recited can be applied to the present disclosure.

### Reagents and Instruments

In the present disclosure, the structure and purity of the compounds were determined by nuclear magnetic resonance (¹H NMR) and/or liquid mass spectrometry (LC-MS).

¹H NMR: Bruker AVANCE-400 nuclear magnetic analyzer, with tetramethylsilane (TMS) as internal standard.

LC-MS: Agilent 1290 HPLC System/6130/6150 MS LC-MS (Manufacturer: Agilent), column Waters BEH/CHS, 50×2.1 mm, 1.7 µm.

Determination of HPLC was performed by Agilent 1260 Infinity HPLC, OpenLAB CDS Chemstation workstation, column XBridge C18 4.6*250 mm, ID 5 µm column, detector DAD.

Elemental analysis was performed using an inductively coupled plasma optical emission spectrometer, model icp 500; power 1300w; flow rate 1 mL/min.

The known starting materials in the following examples may be synthesized using or according to methods known in the art or may be purchased from companies such as ABCR GmbH&Co.KG, Acros Organics, Aldrich Chemical Company, Accela ChemBio Inc, and Darui Chemicals.

As used herein, room temperature in the following examples refers to about 20°C to 30°C.

### General methods

X-ray powder diffraction (XRPD): in the present disclosure, the above crystalline or amorphous powder X-ray diffraction pattern was obtained by a known method in the art using an ARL Equinox3000 X-ray powder diffraction analyzer. The XRPD measurement parameters are shown in Table 5 below.

**Table 5**

| Parameters | XRPD |
|---|---|
| X-ray source | Cu-Kα (λ=1.54056 Angstrom) |
| Light tube setting | 40 Kv, 30 mA |
| Detector | PSD |
| Scan range, 2θ (°) | 0°-108° |
| Scan step, 2θ (°) | 0.05 |
| Scan rate | 1 second/step |

In the X-ray powder diffraction pattern, the position of each peak was determined by 2θ (°). It will be appreciated that different instruments and/or conditions may result in slightly different data being generated and that the position and relative intensity of each peak may vary.

The division of the intensities of the peaks merely reflects the approximate size of the peaks at each position. In the present disclosure, each crystal form has the diffraction peak with the highest peak height as the base peak, and the relative intensity thereof is defined as 100%, as I₀ (a peak with a 2θ (°) value of 18.803 in phosphate crystal form I is the base peak; a peak with a 2θ (°) value of 20.27 in L-tartrate crystal form I is the base peak; a peak with a 2θ (°) value of 21.353 in L-tartrate crystal form II is the base peak; and a peak with a 2θ (°) value of 18.230 in phosphate crystal form II is the base peak), the ratio of the peak height thereof to the peak height of the base peak is taken as the relative intensity I/I₀ of each of the other peaks. The division of the relative intensities of each peak is defined as shown in Table 6 below.

**Table 6**

| Relative Intensity I/I₀ (%) | Definitions |
|---|---|
| 50 to 100 | VS (Very Strong) |
| 25 to 50 | S (Strong) |
| 10 to 25 | M (Medium) |
| 1 to 10 | W(Weak) |

Single crystal X-ray diffraction (SXRD): in the present disclosure, the single crystal X-ray diffraction pattern of the compound of Formula (II) was obtained by a known method in the art using an D8 Venture diffraction analyzer. The SXRD measurement parameters are shown in Table 7 below. After collecting relevant data, the crystal structure was further analyzed by direct method (SHELXT2014) to confirm the absolute configuration.

**Table 7**

| Parameters | SXRD |
|---|---|
| Light source | Cu target |
| X-ray | Cu-Kα (=1.54178 Å) |
| Detector | CMOS Face Detector |
| Current Voltage | 50 kV, 1.2 mA |
| Resolution | 0.80 Å |
| Exposure time | 5 s |
| Test temperature | 170 (2) K |
| Distance from face detector to sample | 40 mm |

An acid-base molar ratio of the phosphate of the compound of Formula (I) disclosed herein and the crystal form thereof was determined by elemental analysis, and the acid-base molar ratio of the L-tartrate of the compound of Formula (I) and the crystal form thereof and the amorphous form thereof was determined by HPLC/IC or ¹H NMR.

High Performance Liquid Chromatography: in the present disclosure, high performance liquid chromatography (HPLC) was collected on an Agilent 1260 HPLC.

Differential Scanning Calorimetry (DSC): in the present disclosure, a differential scanning calorimetry spectrum of the above crystal form was obtained by a known method in the art using a DSC25A differential scanning calorimeter. The DSC measurement parameters are shown in Table 8 below.

**Table 8**

| Parameters | DSC |
|---|---|
| Method | Linear temperature rise |
| Sample disc | Aluminum disc, Open |
| Range of temperatures | 25°C - Set temperature |
| Scan rate (°C/min) | 10 |
| Protection gas | Nitrogen |

Thermogravimetric Analysis (TGA): in the present disclosure, a thermogravimetric analysis spectrum of the above crystal form was obtained by a known method in the art using a TGA550 thermogravimetric analyzer. The TGA measurement parameters are shown in Table 9 below.

**Table 9**

| Parameters | TGA |
|---|---|
| Method | Linear temperature rise |
| Sample disc | Platinum disc, Covered |
| Range of temperatures | 25°C - Set temperature |
| Scan rate (°C/min) | 10 |
| Protection gas | Nitrogen |

Dynamic vapor sorption (DVS) curve: a dynamic vapor sorption curve was collected on a DVS Intrinsic of SMS (Surface Measurement Systems). The relative humidity at 25°C was calibrated using the deliquescent points of LiCl, Mg(NO₃)₂ and KCl. The instrument test conditions are shown in Table 10 below.

**Table 10**

| Parameters | DVS |
|---|---|
| Equilibrium temperature | 25°C |
| Sample quantity | 10 mg to 20 mg |
| Protection gas and flow rate | Nitrogen, 200 mL/min |
| Humidity | 0.00% |
| Maximun equilibrate time | 90.00 min |
| RH range | 0% to 80% to 0% |
| RH gradient | 10% (0% RH to 80% RH, 80% RH to 0% RH) |

Chinese Pharmacopoeia 2020, volume IV, 9103 Guidelines for Hygroscopicity Test of Drugs specifies the definition of hygroscopicity characteristics and hygroscopicity weight gain: (1) deliquescence: absorbing sufficient water to form a liquid; (2) extremely hygroscopicity: hygroscopicity weight gain of not less than 15%; (3) hygroscopicity: hygroscopicity weight gain of less than 15% but not less than 2%; (4) slightly hygroscopicity: hygroscopicity weight gain of less than 2% but not less than 0.2%; (5) no or almost no hygroscopicity: moisture gain is less than 0.2%.

It will be appreciated that additional numerical values may be obtained using other types of instruments having the same function as the instruments described above or using different test conditions than those used in the present disclosure, and accordingly, the numerical values recited are not to be taken as absolute numerical values.

Those skilled in the art can understand that there may be minor differences in the above parameters used to characterize the physical properties of the crystals due to instrumental errors or operator differences and the above parameters are only used to assist in characterizing the polymorphs provided by the present disclosure and are not to be construed as limiting the polymorphs of the present disclosure.

Unless otherwise specified, "phosphoric acid solution" and "L-tartaric acid solution" used in the present disclosure refer to an aqueous solution of phosphoric acid and an aqueous solution of L-tartaric acid. "Phosphate" and "L-tartrate" used in the present disclosure refer to phosphate of the compound of Formula (I) and L-tartrate of the compound of Formula (I).

The solvent used in the present disclosure was an analytically pure solvent, such as ethanol was analytically pure ethanol (water content ≤ 0.3%).

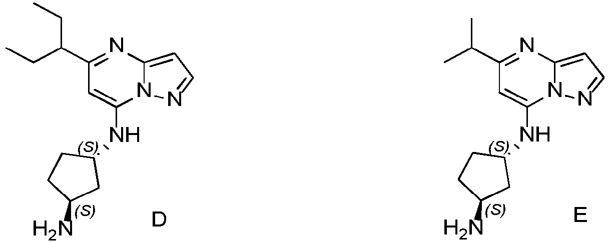

Compound D (CAS: 2416873-83-9) and compound E (CAS: 2416873-60-2) in the present application can be prepared by referring to existing published patent documents.

MTBE refers to methyl tert-butyl ether; DMSO refers to dimethyl sulfoxide; THF refers to tetrahydrofuran; EA refers to ethyl acetate; PE refers to petroleum ether; DCM refers to dichloromethane; MeOH refers to methanol; CDI refers to N, N-carboyldiimidazole; MOPS refers to 3-(N-morpholine)propanesulfonic acid; Tween-20 refers to Tween 20; DTT refers to dithiothreitol; EDTA refers to ethylenediaminetetraacetic acid; CDK1 refers to cell cycle dependent kinase 1; CDK2 refers to cell cycle dependent kinase 2; CDK9 refers to cell cycle dependent kinase 9; K2EDTA refers to dipotassium ethylenediaminetetraacetate; IPA refers to isopropyl alcohol; NH₃ refers to ammonia, as used in the present disclosure.

### Preparation Example 1. Preparation of Compound 6

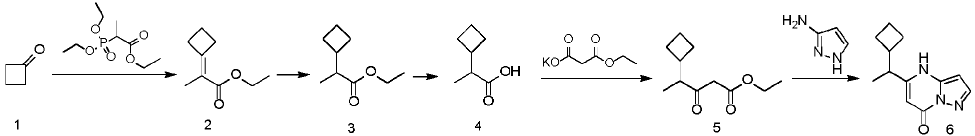

Step 1: Sodium hydride (18 g, 450.00 mmol, purity 60%) was dissolved in THF (300 mL), cooled to 0°C, and triethyl 2-phosphonopropionate (100 g, 419.78 mmol) was slowly added dropwise thereto. The reaction was stirred at 0°C to 5°C for 1 h, and then cyclobutanone (25 g, 356.69 mmol) was dissolved in THF (50 mL) and slowly added dropwise thereto. After the addition was completed, the reaction was carried out at 25°C for 15 h. The reaction was quenched with saturated sodium chloride solution (600 mL), then extracted with ethyl acetate (600 mL×2), and the combined organic phases were washed with saturated sodium chloride solution (500 mL), dried over anhydrous sodium sulfate, filtered, and concentrated under reduced pressure. The residue was separated and purified by CombiFlash (120 g×2, 0% to 20% EA/PE) to obtain Compound **2** (44 g, colorless oil), yield: 80.00%. ¹H NMR (400 MHz, DMSO-*d*₆) δ 4.05 (q, *J*=7.2 Hz, 2H), 3.01-2.93 (m, 2H), 2.78-2.71 (m, 2H), 2.00-1.89 (m, 2H), 1.61-1.58 (m, 3H), 1.18 (t, *J* = 7.2 Hz, 3H).

Step 2: Compound **2** (44 g, 285.33 mmol) was dissolved in methanol (400 mL), and wet palladium carbon (4.4 g, purity 10%) was added thereto. A hydrogen balloon was placed, and the hydrogen was replaced three times. The mixture was stirred at 27°C under a hydrogen atmosphere (15 psi) for 6 to 8 h. TLC showed that the raw material point disappeared. Pd/C was filtered off, and the solvent was removed under reduced pressure to obtain a crude Compound **3** (44 g, colorless oil, boiling point about 182°C), yield: 98.71%, and directly proceeded to the next step without purification.

Step 3: Compound **3** (44 g, 281.65 mmol) was dissolved in methanol (400 mL), sodium hydroxide (45.06 g, 1.13 mol) and water (200 mL) were added thereto, and then the reaction was stirred at room temperature for 16 h. LCMS showed that the reaction was completed. Methanol was removed by concentration under reduced pressure, and then the solution was extracted twice with dichloromethane. The aqueous phase was adjusted to pH 3 with dilute hydrochloric acid (6 M), extracted with DCM (300 mL × 3), and the organic phases were combined and spin-dried to obtain Compound **4** (35 g, colorless oil, boiling point of about 220°C), yield: 96.96%, and directly proceeded to the next step without purification. MS m/z (ESI):127.1 [M-H]⁻. ¹H NMR (400 MHz, DMSO-*d*₆) δ 11.91 (s, 1H), 2.36-2.20 (m, 2H), 1.99-1.90 (m, 2H), 1.82-1.58 (m, 4H), 0.93 (d, *J =* 6.8 Hz, 3H).

Step 4: Compound **4** (36 g, 280.88 mmol) was dissolved in THF (300 mL) in a reaction flask, and then CDI (68.32 g, 421.32 mmol) was added thereto, and then the solution was reacted at room temperature for 16 h (as solution A). Potassium monomethyl malonate (143.42 g, 842.64 mmol) was added to anhydrous magnesium chloride (66.86 g, 702.20 mmol) and THF (900 mL) in another reaction flask, and the solution was heated at 50°C under argon protection for 16 h (as solution B). Then, the solution A was added dropwise to the solution B at room temperature (about 10 min), and the mixed solution was stirred at 30°C for 16 h. LCMS detected that the reaction was completed and the product was produced. 800 mL of water was added to the reaction solution, and the solution was extracted with ethyl acetate (800 mL × 3). The organic phases were combined and washed with brine, dried over anhydrous sodium sulfate, and then dried under reduced pressure. The residue was separated and purified by CombiFlash (120 g × 2, 0% to 15% EA/PE) to obtain the product Compound **5** (42g, colorless oil), yield: 75.42%. MS m/z (ESI):199.1 [M+H]⁺. ¹H NMR (400 MHz, DMSO-*d*₆) δ 4.13-4.00 (m, 2H), 3.55 (d, *J* = 0.8 Hz, 2H), 2.63-2.54 (m, 1H), 2.42-2.28 (m, 1H), 1.97-1.85 (m, 2H), 1.83-1.74 (m, 1H), 1.73-1.61 (m, 3H), 1.16 (t*, J =* 7.2 Hz, 3H), 0.91 (d, *J =* 6.8 Hz, 3H).

Step 5: Compound **5** (42 g, 211.85 mmol) and 3-aminopyrazole (19.36 g, 233.03 mmol) were dissolved in glacial acetic acid (300 mL), and the temperature was raised to 120°C for reaction for 16 h. LCMS showed that the reaction was completed. The acetic acid was removed by concentration under reduced pressure, and the mixture was slurried with ethyl acetate (800 mL × 4) to precipitated a solid, filtered, and dried to obtain Compound **6** (41 g, light yellow solid), yield: 89.08%. The compound was directly proceeded to the next step without purification. MS m/z (ESI):218.1 [M+H]⁺.

### Preparation Example 2. Preparation of the compound of Formula (I)

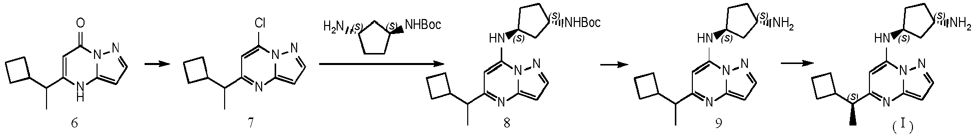

Step 1: Compound 6 (473.6 mg, 2.18 mmol) was dissolved in phosphorus oxychloride (6 mL), and the solution was heated to 120°C and stirred for 3 hours. After cooling to room temperature, the solution was poured into ice water (60 g) and extracted with dichloromethane (80 mL). The organic phase was dried over anhydrous sodium sulfate, filtered, and concentrated under reduced pressure. The residue was separated by silica gel column chromatography (20 g, 0% to 40% EA/DCM) to obtain Compound 7. MS m/z (ESI):236.1 [M+H]⁺; ¹H NMR (400 MHz, DMSO-*d₆)* δ 8.25 (d, J = 2.4 Hz, 1H), 7.35 (s, 1H), 6.76 (d, J = 2.4 Hz, 1H), 2.893-2.84 (m, 1H), 2.61-2.51 (m, 1H), 2.12-2.01 (m, 1H), 1.80-1.59 (m, 5H), 1.14 (d, J = 6.8 Hz, 3H).

Step 2: Compound 7 (142.7 mg, 605.5 µmol) and tert-butyl ((1S, 3S)-3-aminocyclopentyl)carbamate (121.27 mg, 605.52 µmol) were dissolved in acetonitrile (20 mL), and then potassium carbonate (251.0 mg, 1.81 mmol) was added thereto. The reaction was stirred at 90°C for 16 h. Ethyl acetate (80 mL) was added for dilution, and the solution was washed with saturated sodium chloride solution (80 mL×3). The organic phase was dried over anhydrous sodium sulfate, filtered, and concentrated under reduced pressure to obtain Compound **8.** MS m/z (ESI):400.3 [M+H]⁺; ¹H NMR (400 MHz, DMSO-*d₆)* δ 8.00 (d, J = 2.0 Hz, 1H), 7.58 (d, J = 7.6 Hz, 1H), 6.97 (d, J = 7.6 Hz, 1H), 6.30 (d, J = 2.0 Hz, 1H), 6.00 (s, 1H), 4.19 (q, J = 7.2 Hz, 1H), 4.02 - 3.93 (m, 1H), 2.77-2.66 (m, 1H), 2.61-2.53 (m, 1H), 2.21-2.01 (m, 3H), 1.97-1.88 (m, 2H), 1.80-1.63 (m, 6H), 1.56-1.42 (m, 1H), 1.40 (s, 9H), 1.13 (d, J = 6.8 Hz, 3H).

Step 3: Compound **8** (174.7 mg, 437.38 µmol) was dissolved in 1,4-dioxane (3 mL), and hydrochloric acid solution (3.0 mL, 4 M) was added thereto. The solution was stirred at room temperature for 3 h. The solvent was evaporated under reduced pressure, water (60 mL) was added thereto, and the solution was extracted with ethyl acetate (50 mL). The aqueous phase was adjusted to pH = 9 to 10 with saturated sodium carbonate solution, and extracted with ethyl acetate (60 mL×2). The organic phase was dried over anhydrous sodium sulfate, filtered, and concentrated under reduced pressure. Compound **9** was separated by preparative HPLC chromatography. MS m/z (ESI): 300.2 [M+ H]⁺; ¹H NMR (400 MHz, DMSO-*d₆*) δ 7.97 (s, 1H), 7.54 (s, 1H), 6.28 (s, 1H), 5.99 (s, 1H), 4.13-4.22 (m, 1H), 3.90-3.99 (m, 1H), 2.75-2.6.3 (m, 1H), 2.51-2.57 (m, 1H), 2.20-1.96 (m, 3H), 1.85-1.94 (m, 2H), 1.60-1.78 (m, 6H), 1.40-1.49 (m, 1H), 1.11 (d, J = 6.8 Hz, 3H).

Step 4: Compound **9** (94.46 mg, 315.48 µmol) was subjected to chiral separation (column type: IC-3 4.6*100 mm 3 µm; co-solvent: IPA[1% NH₃(7 M in MeOH)]; sample volume: 5.00 µL; wavelength: 220.0nm; running time: 6.0 min; flow rate: 3.0 mL/min; pressure: 2000 psi; column temperature: 40°C) to obtain the compound of Formula (I) (9.70 mg, retention time 2.471 min, yield: 9.89%, purity: 96.34%. MS m/z (ESI): 300.2 [M+H]⁺. ¹H NMR (400 MHz, DMSO-*d₆*) δ 7.96 (d, J = 2.0 Hz, 1H), 7.41 (d, J = 7.6 Hz, 1H), 6.27 (d, J = 2.0 Hz, 1H), 5.96 (s, 1H), 4.21 (q, J = 7.2 Hz, 1H), 3.42 (q, J = 6.0 Hz, 1H), 2.65-2.74 (m, 1H), 2.58-2.50 (m, 1H), 2.16-2.24 (m, 1H), 2.03-2.09 (m, 1H), 1.83-1.95 (m, 2H), 1.80-1.61 (m, 7H), 1.35-1.25 (m, 1H), 1.11 (d, J = 6.8 Hz, 3H).

The compound of Formula (II) was prepared from the compound of Formula (I), so that the absolute configuration of the compound of Formula (I) can be determined by the absolute configuration of the compound of Formula (II).

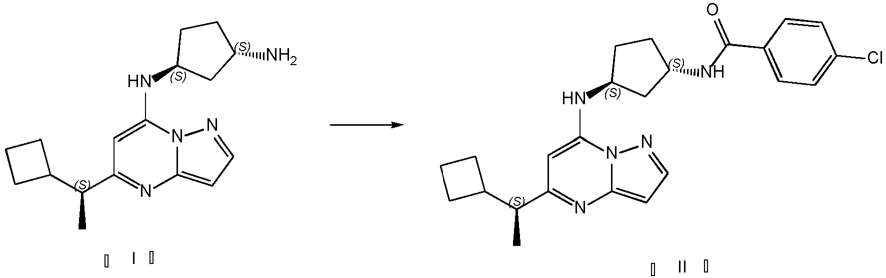

The compound of Formula (I) (200 mg, 667.97 µmol) was dissolved in dichloromethane (10 mL), and then 4-chlorobenzoyl chloride (175.4 mg, 1.00 mmol) was added thereto, followed by the addition of N,N-diisopropylethylamine (0.6 mL, 3.34 mmol). The reaction was stirred at room temperature for 2 h, and LCMS detected that the raw material was reacted completely. Water (30 mL) was added thereto, and the solution was extracted with ethyl acetate (30 mL). The organic phase was washed with saturated sodium chloride solution (30 mL × 3), dried over anhydrous sodium sulfate, filtered, and concentrated under reduced pressure to obtain a crude product. The crude product was separated and purified by silica gel column chromatography to obtain the compound of Formula (II) (160 mg, yield: 54.69%). LCMS (ESI) m/z: 438.2 [M + H]⁺.

The single crystal of the compound of Formula (II) was further prepared, and the ellipsoid graph of the molecular stereostructure of a single crystal of the compound of Formula (II) is shown in FIG. 9. The Flack constant is 0.07(2), C8, C10 and C19 are S configuration, that is, the absolute configuration of the three chiral centers of the compound of Formula (II) is S configuration, and the structure is shown in the structure of the compound of Formula (II). Therefore, it can be determined that the absolute configuration of the three chiral centers of the compound of Formula (I) is S configuration, and its structure is shown in the structure of the compound of Formula (I).

### Example 1. Preparation of the phosphate crystal form I of the compound of Formula (I)

Free base (500 mg) and methanol (1 mL) were added into a 20 mL sample bottle, dissolved by sonication, and then 1 mol/L phosphoric acid solution was added thereto according to the molar ratio of feed of acid to base =2.5: 1. The reaction was carried out at 50°C for 1 h, and stirring was continued at 40 °C for 1 h, and then the stirring was continued at 30°C for another 1 h. Finally, the heating was turned off and the solution was stirred overnight. After the reaction was completed, the temperature was slowly cooled to 0°C, acetone was added to the solution to precipitate solids, the solids were collected by centrifugation and the solvent was evaporated to obtain the phosphate crystal form I of the compound of Formula (I). The phosphorus content of the obtained solid was 12.5% determined by elemental analysis, indicating that the molar ratio of the compound of Formula (I) to phosphoric acid in the obtained solid was 1:2 (the theoretical value of phosphorus content thereof was 12.5%); the XRPD pattern of the obtained solid was substantially as shown in FIG. 1, and the XRPD pattern of the obtained solid has peaks at the 2θ (°) values as shown in the aforementioned Table 1, and the relative intensities of each peak are shown in the aforementioned Table 1; the DSC and TGA spectrums thereof are shown in FIG. 2, the DVS spectrum thereof is shown in FIG. 3, and the microscope image thereof is shown in FIG. 4.

As shown in FIG. 2, in the DSC spectrum, there is a melting absorption peak at 188.01°C, and the crystallinity is good, indicating that the melting point of the phosphate crystal form I of the compound of Formula (I) is about 188.01°C; in the TGA spectrum, the sample loses 1.139% of its weight when heated to around 190°C, which may be due to the volatilization of solvent.

As shown in FIG. 3, in the DVS spectrum, under 80% RH conditions, the weight gain due to moisture absorption is 9%, indicating that the phosphate crystal form I of the compound of Formula (I) has hygroscopicity.

As shown in FIG. 4, the polarizing microscope image shows that the phosphate crystal form I of the compound of Formula (I) is rod-shaped and block-shaped.

### Example 2. Preparation of the L-tartrate crystal form I of the compound of Formula (I)

Free base (500 mg) and alcohol (1 mL) were added into a 20 mL sample bottle, dissolved by sonication, and then 1 mol/L L-tartaric acid solution was added thereto according to the molar ratio of feed of acid to base =1.2:1. The reaction was carried out at 50°C for 1 h, and stirring was continued at 40 °C for 1 h, and then the stirring was continued at 30°C for another 1 h. Finally, the heating was turned off and the solution was stirred at room temperature overnight. After the reaction was completed, the temperature was slowly cooled to 0°C, the solid was collected by centrifugation and the solvent was evaporated. The obtained solid is L- tartrate crystal form I of the compound of Formula (I). The HPLC/IC results showed that the molar ratio of the compound of Formula (I) to L-tartaric acid in the obtained solid was 1:1; the XRPD pattern was substantially as shown in FIG. 5, and the XRPD pattern of the obtained solid has peaks at the 2θ (°) values as shown in the aforementioned Table 2, and the relative intensities of the peaks are shown in the aforementioned Table 2; the TGA spectrum is shown in FIG. 6.

As shown in FIG. 6, the TGA spectrum shows that the weight loss is 1.057% when heated to around 110°C, which may be due to the volatilization of water, and the weight loss continues to be 3.482% when heated to around 178°C, which is presumably due to the volatilization of organic solvents, and melting decomposition occurs when heated continuously.

### Example 3. Preparation of the L-tartrate crystal form II of the compound of Formula (I)

Free base (100 mg) and methanol (0.2 mL to 0.3 mL) were added into a sample bottle, dissolved by sonication, and then 1 mol/L L-tartaric acid solution was added thereto according to the molar ratio of feed of acid to base =1.2:1. The reaction was carried out at 50°C for 1 h, and stirring was continued at 40°C for 1 h, and then the stirring was continued at 30°C for another 1 h. After cooling to room temperature, the heating was turned off and the solution was stirred overnight. After the reaction was completed, the temperature was slowly cooled to 0°C, MTBE was added to the solution to crystallize, and the solvent was evaporated to obtain solids. The obtained solid was subjected to HPLC/IC detection to determine that the molar ratio of the compound of Formula (I) to L-tartaric acid in the obtained solid was 1:1; the XRPD pattern of the obtained solid is shown in FIG. 7, and the XRPD pattern of the obtained solid has peaks at the 2θ (°) values as shown in the aforementioned Table 3, and the relative intensities of the peaks are shown in Table 3. It is defined in the present disclosure as L-tartrate crystal form II of the compound of Formula (I).

### Example 4. Preparation of the amorphous L-tartrate of the compound of Formula (I)

Free base (100 mg) and acetone (0.2 mL to 0.3 mL) were added into a sample bottle, dissolved by sonication, and then 1 mol/L L-tartaric acid solution was added thereto according to the molar ratio of feed of acid to base =1.2:1. The reaction was carried out at 50°C for 1 h, and stirring was continued at 40°C for 1 h, and then the stirring was continued at 30°C for another 1 h. After cooling to room temperature, the heating was turned off and the solution was stirred overnight. After the reaction was completed, the temperature was slowly cooled to 0°C to precipitate a solid, the solid was collected by centrifugation and the solvent was evaporated. The obtained solid was subjected to HPLC/IC to determine that the molar ratio of the compound of Formula (I) to L-tartaric acid in the obtained solid was 1:1; the XRPD pattern of the obtained solid is shown in FIG. 8, which is defined as the amorphous L-tartrate in the present application.

### Example 5. Preparation of the phosphate crystal form II of the compound of Formula (I)

The free base (100 mg) in a 50 mL sample bottle was dissolved in ethanol (15 mL), and then an ethanol solution of phosphoric acid (5 ml) was added dropwise thereto according to the molar ratio of feed of acid to base =0.95:1, stirred at room temperature for 4 h, filtered and rinsed with ethanol, and dried by rotary evaporation to obtain a white solid. The obtained solid was analyzed by elemental analysis to obtain a phosphorus content of 7.8%, indicating that the molar ratio of the compound of Formula (I) to phosphoric acid in the obtained solid was 1:1 (the theoretical value of phosphorus content thereof was 7.79%); the obtained solid was subjected to XRPD detection, and its XRPD pattern is shown in FIG. 10, which is defined as the phosphate crystal form II in the present application.

The XRPD pattern of the obtained solid has peaks at the 2θ (°) values as shown in the aforementioned Table 4; and the relative intensities of each peak are shown in the aforementioned Table 4; the DSC and TGA curves thereof are shown in FIG. 11, the DVS spectrum thereof is shown in FIG. 12, and the microscope image thereof is shown in FIG. 13.

As shown in FIG. 11, in the DSC curve, the sample has a melting absorption peak at 207.48°C, and the crystallinity is good, indicating that the melting point of the phosphate crystal form II of the compound of Formula (I) is about 207.48°C; in the TGA curve, the sample has almost no weight loss when heated to around 100°C, and begins to melt when heated to around 207°C. Therefore, the phosphate crystal form II of the compound of Formula (I) is an anhydrous substance.

As shown in FIG. 12, in the DVS spectrum, under 80% RH conditions, the weight gain due to moisture absorption is 0.45%, indicating that the phosphate crystal form II of the compound of Formula (I) has slight hygroscopicity.

As shown in FIG. 13, the polarizing microscope image shows that the phosphate crystal form II of the compound of Formula (I) is rod-shaped.

### Example 6. Preparation of the phosphate crystal form II of the compound of Formula (I)

The free base (100 mg) in a 50 mL sample bottle was dissolved in acetone (15 mL), and then an ethanol solution of phosphoric acid (5 ml) was added dropwise thereto according to the molar ratio of feed of acid-base =0.98:1, stirred at room temperature for 4 h, filtered and rinsed with ethanol, and dried by rotary evaporation to obtain a white solid. The phosphorus content of the obtained solid was 7.8% determined by elemental analysis, indicating that the molar ratio of the compound of Formula (I) to phosphoric acid in the obtained solid was 1:1 (the theoretical value of phosphorus content thereof was 7.79%); the obtained solid was subjected to XRPD detection, and its XRPD pattern was substantially as shown in FIG. 10.

### Example 7. Preparation of the phosphate crystal form II of the compound of Formula (I)

The free base (100 mg) in a 50 mL sample bottle was dissolved in ethanol (10 mL), and then an ethanol solution of phosphoric acid (10 ml) was added dropwise thereto according to the molar ratio of feed of acid-base =1.05:1, stirred at room temperature for 4 h, filtered and rinsed with ethanol, and dried by rotary evaporation to obtain a white solid. The phosphorus content of the obtained solid was 7.8% determined by elemental analysis, indicating that the molar ratio of the compound of Formula (I) to phosphoric acid in the obtained solid was 1:1 (the theoretical value of phosphorus content thereof was 7.79%); the obtained solid was subjected to XRPD detection, and its XRPD pattern was substantially as shown in FIG. 10.

### Example 8. Preparation of the phosphate crystal form II of the compound of Formula (I)

0.5 g of free base was dissolved in methanol (15 ml), phosphoric acid (commercially available) was added thereto according to the molar ratio of feed of acid to base =1:1, and the methanol was removed by rotary evaporation after stirring for 2 h to obtain a crude phosphate. Ethanol (2.5 ml) was added to the solution and the solution was concentrated, and then ethanol (2.5 ml) was added to the solution and the solution was concentrated again. Ethanol (10 ml) was added to the solution and the solution was heated to 60°C. After stirring for 3 h, the solution was cooled to room temperature and stirred overnight. The solution was filtered, rinsed with ethanol, and dried by rotary evaporation to obtain phosphate form II of the compound of Formula (I) with a yield of 75.8%. The phosphorus content of the obtained solid was 7.7% determined by elemental analysis, indicating that the molar ratio of the compound of Formula (I) to phosphoric acid in the obtained solid was 1:1 (the theoretical value of phosphorus content thereof was 7.79%); the obtained solid was subjected to XRPD detection, and its XRPD pattern was substantially as shown in FIG. 10.

### Comparative Example 1. Salt-forming reaction of the compound of Formula (I)

The compound of Formula (I) (100 mg) was weighed by weight reduction method into a transparent sample bottle, and corresponding solvent (0.2 mL to 0.3 mL) was added to the transparent sample bottle, dissolved by sonication, and then 1 mol/L acid solution (aqueous solution) was added thereto according to the molar ratio of feed of acid to base =1.2:1. The reaction was carried out at 50°C for 1 h, and stirring was continued at 40°C for 1 h, and then the stirring was continued at 30°C for another 1 h. After cooling to room temperature, the heating was turned off and the solution was stirred overnight. After the reaction was completed, the temperature was slowly cooled until the solid was precipitated. If it was still a clear solution, try to induce crystallization by adding an anti-solvent. The reaction results were sent to XRPD for testing. The results show that hydrochloric acid, sulfuric acid, oxalic acid and other acids cannot salify the compound of Formula (I) after cooling and adding an anti-solvent; the types of acids and solvents are shown in Table 11 below.

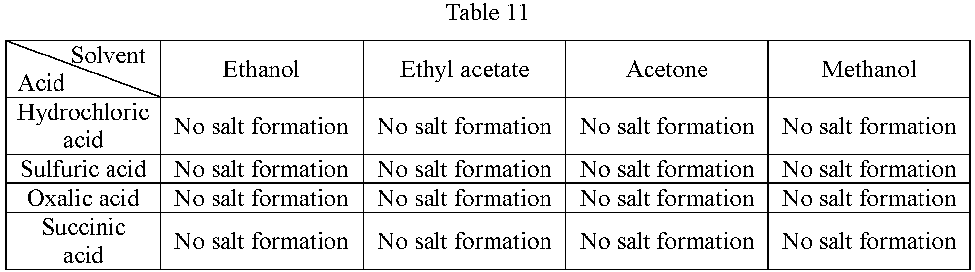

### Test Example 1. Solubility Test

The solubility of the phosphate of the compound of Formula (I) and L-tartrate of the compound of Formula (I) prepared in the above Examples in water was tested at room temperature. The solubility of the phosphate was no more than 26.6 mg/mL, the solubility of the L-tartrate was no more than 17.3 mg/mL, and the free base was slightly soluble in water. The salt of the compound of Formula (I) greatly improves the solubility thereof in water, and the phosphate salt has a higher solubility than the L-tartrate.

In addition, the rough solubility (unit: mg/mL) of the phosphate crystal form I and L-tartrate crystal form I of the compound of Formula (I) in a pH 4.5 buffer (acetic acid-sodium acetate system) and a pH 6.8 buffer (sodium dihydrogen phosphate-sodium hydroxide system) was tested at room temperature, and the results are shown in Table 12. From the results in Table 12, it can be seen that the phosphate crystal form I and L-tartrate crystal form I of the compound of Formula (I) have high solubility in the buffers of pH 4.5 and pH 6.8.

**Table 12**

| Samples to be tested | Solubility in pH 4.5 buffer (mg/mL) | Solubility in pH 6.8 buffer (mg/mL) |
|---|---|---|
| Phosphate crystal form I | ≤33 | ≤24.5 |
| L-tartrate crystal form I | ≤43.3 | ≤21.8 |

An appropriate amount of phosphate crystal form I or phosphate crystal form II was weighed, and 0.1 mol/L hydrochloric acid solution (5 mL) and pH 6.8 phosphate buffer (5 mL) were added to the mixture, the samples were taken at 24 h (placed in an oven at 37°C) to determine the solubility. The results are shown in Table 13.

**Table 13**

| Item | | Solubility (mg/ml) |
|---|---|---|
| Phosphate crystal form I | 0.1N HCl | 22.54 |
| | pH 6.8 | 22.46 |
| Phosphate crystal form II | 0.1N HCl | 39.14 |
| | pH 6.8 | 33.63 |

### Test Example 2. Stability Test

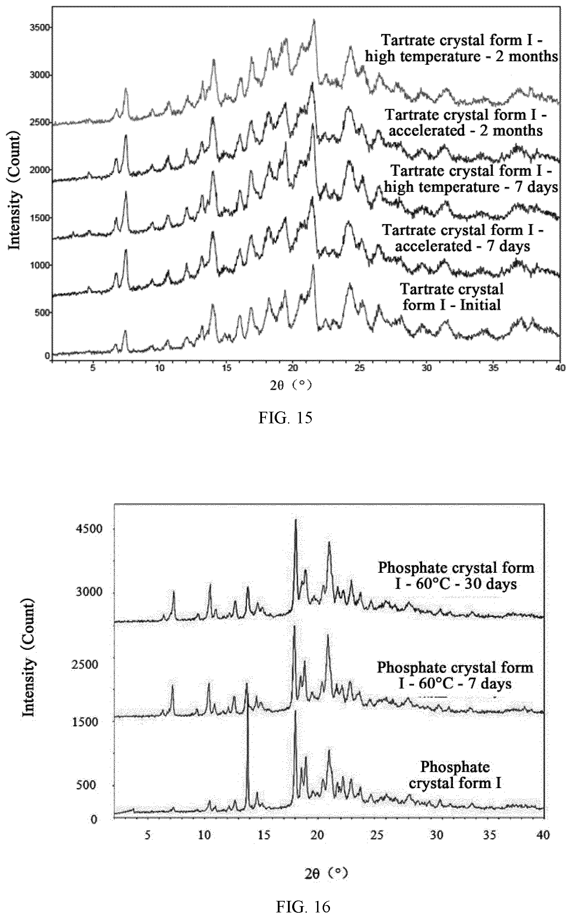

(1) Samples (about 100 mg) of phosphate crystal form I of the compound of Formula (I), phosphate crystal form II of the compound of Formula (I), L-tartrate crystal form I of the compound of Formula (I) and L-tartrate crystal form II of the compound of Formula (I) were weighed, respectively, and placed at 60°C (high temperature) and 40°C to 75% RH (accelerated), while another group of samples were sealed and stored at 5°C as controls. The changes in crystal form were detected at day 7, day 20, day 30, and day 60, respectively.
The test results show that the crystal forms of the L-tartrate form I of the compound of Formula (I), the L-tartrate form II of the compound of Formula (I), the phosphate form I of the compound of Formula (I), and the phosphate form II of the compound of Formula (I) have no obvious changes under the above conditions and have high stability. The crystal form I of the L-tartrate of the compound of Formula (I) was stored under high temperature conditions for 7 days and 2 months (60 days), the XRPD pattern thereof showed no obvious change; the overlay of the XRPD patterns of the crystal form I of the L-tartrate of the compound of Formula (I) stored under accelerated conditions for 7 days and 2 months (60 days) is shown in FIG. 15, showing no obvious change; this indicates that the crystal form I of the L-tartrate of the compound of Formula (I) has high stability. The phosphate crystal form I of the compound of Formula (I) was stored under high temperature conditions for 7 days and 30 days, and the overlay of the XRPD patterns of its crystal form is shown in Figure 16, showing no obvious change, indicating that the phosphate crystal form I of the compound of Formula (I) has high stability.
(2) About 40 mg of phosphate crystal form I (prepared with reference to Example 1 of the present application) was weighed into a glass vial , and ethanol (1 mL) was added thereto, the glass vial was sealed and shaken at 50°C and 150 r/min for seven days, then centrifuged to collect the solid and the solvent was evaporated. The obtained solid was performed XRPD detection, and the XRPD pattern thereof is shown in FIG. 10. The obtained solid is phosphate crystal form II. The test results in FIG. 14 show that phosphate crystal form I can be transformed into phosphate crystal form II which is more thermodynamically stable.
(3) 25 mg of phosphate crystal form I and phosphate crystal form II were weighed, respectively, and 5 mg of water was added thereto respectively, and then the samples were taken in an oven at 80°C for one week. Samples were taken and the related substances were determined. The results are shown in Table 14, indicating that phosphate crystal form I and phosphate crystal form II have high chemical stability.

**Table 14**

| Group | Phosphate content (0 d) | Phosphate content (80°C, 7 d) |
|---|---|---|
| Phosphate crystal form I | 99.64 | 98.66 |
| Phosphate crystal form II | 99.64 | 98.73 |

### Test Example 3. Hygroscopicity Test

### 3.1 Hygroscopicity test of L-tartrate

The hygroscopicity test was carried out according to the Guidelines for Hygroscopicity Test of Drugs (Chinese Pharmacopoeia 2020, volume IV, 9103), and it was found that the L-tartrate of the compound of Formula (I) has slight hygroscopicity.

### 3.2 Hygroscopicity test of phosphate crystal form

DVS measurements were performed on the phosphate crystal form I and the phosphate crystal form II of the compound of Formula (I). The DVS spectrum of the phosphate crystal form I of the compound of Formula (I) is shown in FIG. 3. The DVS spectrum shows that under the condition of 80% RH, the weight gain of the phosphate crystal form I of the compound of Formula (I) has hygroscopicity. The DVS spectrum of the phosphate crystal form II shows that under the condition of 80% RH, the weight gain of the phosphate crystal form II is only 0.45%, indicating that the phosphate crystal form II has slight hygroscopicity.

### Test Example 4. Activity inhibition test on CDK family kinases

In the following LANCE Ultra test method, kinase reagents were purchased from Carna Bioscience, reaction substrates and detection reagents were purchased from Perkin Elmer, and the remaining reagents were purchased from Thermo scientific.

The LANCE Ultra method was used to determine the inhibitory effect of the test substance on the kinase activity of CDK1/CycB (Carna bioscience, #04-102), CDK2/ CycA (Carna bioscience, #04-103), CDK9/CycT (Carna bioscience, #04-110).

The kinase activity was tested using a 10 µL system, comprising the following components: CDK kinase dilution, substrate diluent mixed with Ulight-Myelicbasicprotein (PerkinElmer, #TRF-0109, hereinafter referred to as U-MBP) and ATP (Thermoscientific, #PV3227), and the compound of Formula (I) disclosed in the present disclosure (i.e., the test substances). Each kinase in the test included three test groups: background group (Blank), non-inhibition group (PC) and compound test group (Test). The components comprised in each test group are shown in Table 15 below.

**Table 15**

| | Kinase | Substrate | Compound |
|---|---|---|---|
| Blank | 1.33x Reaction buffer | Substrate dilution | Only 2% DMSO |
| PC | Kinase dilution | Substrate dilution | Only 2% DMSO |
| Test | Kinase dilution | Substrate dilution | Compound dilution |

The working concentrations of the components of the Test group in different kinase reactions are shown in Table 16:
Compound: 10 mM of the compound to be tested were dissolved at room temperature and diluted in DMSO gradients, followed by 4x compound working solutions diluted with deionized water at 2% DMSO. The highest concentrations of compound used in CDK1 and CDK2 assays were 10 µM and in CDK9 was 1 µM.

1.33x Reaction buffer: Composed of 26.7 mM MOPS, 6.67 mM MgCl₂ and 0.0133% Tween-20, stored in refrigerator at 4°C and protected from light after preparation. Freshly prepared DTT was added to the final concentration of 5.33 mM before use.

**Table 16**

| Components | Kinase | U-MBP | ATP | 4x Compound |
|---|---|---|---|---|
| Solvent | 1.33x reaction buffer | 1.33x reaction buffer | | 2% DMSO |
| Volume | 2.5 µL | 5 µL | | 2.5 µL |
| CDK1 | 0.1 ng/µL | 12.5 nM | 4 µM | gradient dilution |
| CDK2 | 0.25 ng/µL | 12.5 nM | 10 µM | gradient dilution |
| CDK9 | 1.25ng/µL | 12.5 nM | 10 µM | gradient dilution |

The DMSO working concentration in the reaction was 0.5%.

The above components were mixed and incubated on a shaker for 1 h at room temperature, protected from light. Subsequently, 10 µL of test solution was added to all test groups (including Blank, PC and Test groups).

10 µL of detection solution comprises the following components: 16 mM EDTA (Thermo scientific, #15575), 1 nM phosphorylated U-MBP protein antibody (PerkinElmer, #TRF-0201) and 1x detection buffer (PerkinElmer, #CR97-100).

After the addition of the test solution, it was placed on a shaker and incubated at room temperature for 1 h in the dark. At the end of the incubation, the signal was read using a VictorX5 fluorescence microplate reader from PerkinElmer, excitation wavelength was 320 nm, emission wavelengths were 615 nm and 665 nm, and the inhibition rate was calculated as follows:
1. The 665 nm/615 nm values (hereinafter referred to as the Ratio value) were calculated for all groups, and the inhibition rate was calculated based on the Ratio value of each group;
2. Suppression ratio = (PC_{Ratio}-Test_{Ratio})/(PC_{Ratio}-Blank_{Ratio})*100%;
3. XLFIT 5.0 software (IDBS, UK) was used for fitting, with logarithmic values of compound concentrations as the X axis and inhibition rates as the Y axis, and IC₅₀ values of compounds were calculated using a four-parameter model. The results are shown in Table 17.

**Table 17**

| Compound | CDK9 (IC₅₀/µM) | CDK1 (IC₅₀/µM) | CDK2 (IC₅₀/µM) |
|---|---|---|---|
| The compound of Formula (I) | 0.012 | 1.887 | 3.577 |

As can be seen from the results in Table 17, the compounds of Formula (I) of the present disclosure have high inhibitory activity against CDK9 and high CDK9 inhibitory selectivity.

### Test Example 5: In vivo pharmacokinetic test in mice

LC/MS/MS method was used to determine the plasma concentrations of Compound D, Compound E and compound of Formula (I) in mice at different time points after intravenous injection and intragastric administration, respectively. The pharmacokinetic behavior of Compound D, Compound E and compound of Formula (I) in mice was studied and pharmacokinetic characteristics thereof were evaluated.

### Experimental protocol:

Test animals: healthy adult male ICR mice (weighing 30-40 g, 12 mice, intravenous injection group mice drinking water and diet ad libitum, gavage group fasting overnight, drinking water and diet ad libitum 4 h after administration), provided by Beijing Vital River Laboratory Animal Co. LTD;

Administration method and dosage: ICR mice were administered via caudal vein administration (2 mg/kg, 5% DMSO, pH 4.5 20% Captisol) and gavage administration (10 mg/kg, 5% DMSO, pH 4.5 20% Captisol).

Blood sampling: Animals meeting the experimental requirements were selected prior to administrating, weighed and labeled. Mice were bound prior to blood sampling, and each administrated mice was bled at a predetermined time point (caudal vein administration: blood samples were collected at 0.083 h, 0.25 h, 0.5 h, 1 h, 2 h, 4 h, 6 h, 7.5 h and 24 h after administration, totally 9 time points; gavage administration: blood samples were collected at 0.083 h, 0.25 h, 0.5 h, 1 h, 2 h, 4 h, 6 h, 7.5 h and 24 h after administration, totally 9 time points), and about 100 µL blood samples were collected through the orbit. The blood was transferred to a 1.5 mL test tube pre-added with K2EDTA, centrifuged for 4 min (8000 rpm, 4°C), and the plasma was removed. The whole process was completed within 15 min after blood collection. All samples were stored in a refrigerator at -20°C until sample analysis. LC/MS/MS method was used to determine drug concentrations. Pharmacokinetic parameters of Compound D, Compound E and the compound of Formula (I) in mice at the same dose and mode of administration are shown in Table 18.

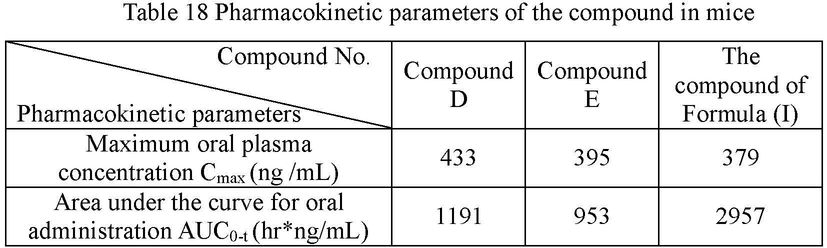

### Test Example 6: In vivo pharmacodynamic experiment

In vivo pharmacodynamic experiments were performed on BALB/c nude mice subcutaneously implanted with MV4 -11 acute myeloid leukemia patient-derived xenograft (CDX) based on human tumor cell lines.

Protocol: BALB/c nude mice, female, 6-10 weeks old, weighing approximately 20-23 grams, were maintained in a special pathogen-free environment and in individual ventilated cages (5 mice per cage, 2 cages of 10). All cages, bedding and water were disinfected prior to use. All animals had free access to standard certified commercial laboratory diets. A total of 80 animals were purchased from the Laboratory Animal Management Department of Shanghai City Institute of Family Planning Science (No. 3577 Jinke Road, Pudong, Shanghai) were used for the study. Each mouse was implanted with tumor cells subcutaneously in the right flank (1×10⁷ 0.1 ml+Matrigel 0.1 ml) for tumor growth. When the average tumor volume reached about 165 cubic mm, randomization was performed according to body weight and tumor volume, and administration was started. The test compound was administered orally by gavage daily. Antitumor efficacy was determined by dividing the average tumor increase volume of animals treated with the compound by the average tumor increase volume of untreated animals.

Tumor volume was measured twice weekly with a two-dimensional caliper, and volumes were measured in cubic millimeters. Tumor volume TV=0.5a×b². Wherein, a is the long diameter of the tumor and b is the short diameter of the tumor.

T/C(%) is the percentage of the relative tumor volume (RTV) between the treatment group and the control group at a certain time point. The calculation Formula is as follows: T/C% = T_{RTV}/C_{RTV}×100% (T_{RTV}: mean RTV of treatment group; C_{RTV}: mean RTV of vehicle control group; RTV=Vₜ/V₀, V₀ is tumor volume of the animal at grouping, Vₜ is tumor volume of the animal after treatment). mpk is mg/kg body weight.

Body weight change (%) of tumor-bearing animals was calculated as follows: (body weight at measurement - body weight at grouping)/body weight at grouping × 100.

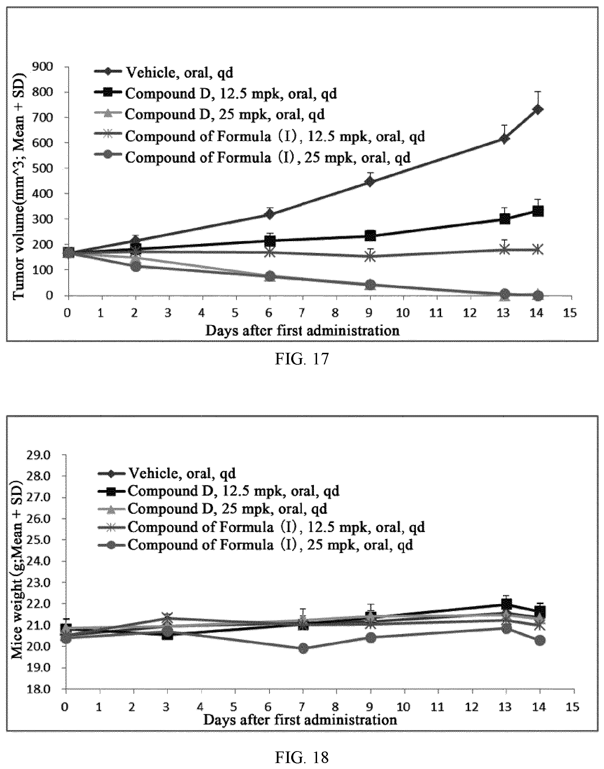

The tumor volume change graph and the mouse body weight change graph after once daily (qd) oral administration of Compound D and the compound of Formula (I) are shown in FIGS. 17 and 18, respectively, and the tumor inhibition results of the compound in mice are shown in Table 19:

**Table 19 Tumor inhibition results of compounds in mice**

| Compound No. | Dosage and mode of administration | Mean tumor weight (g) | SEM | T/C (%) |
|---|---|---|---|---|
| Vehicle group | Oral, Once daily | 0.79 | 0.13 | - |
| Compound D | 12.5 mpk, Oral, Once daily | 0.35 | 0.09 | 45.04 |
| The compound of Formula (I) | 12.5 mpk, Oral, Once daily | 0.17 | 0.06 | 21.50 |
| Compound D | 25 mpk, Oral, Once daily | 0.02 | 0.08 | 2.04 |
| The compound of Formula (I) | 25 mpk, Oral, Once daily | 0.01 | 0.00 | 1.40 |

All documents mentioned in the present disclosure are cited as references in the present application, just as each document is cited as reference individually. In addition, it should be understood that after reading the above teachings of this disclosure, those skilled in the art may make various changes or modifications to the present disclosure, and these equivalent forms also fall within the scope defined by the claims attached to the present application.

The technical features of the above-described embodiments may be arbitrarily combined. To make the description concise, not all possible combinations of the technical features in the above-described embodiments are described. However, as long as there is no contradiction in the combination of these technical features, they should be considered to be within the scope of the description.

The above-described embodiments only express several implementation methods of the present disclosure, and the descriptions thereof are relatively specific and detailed, but they cannot be understood as limiting the scope of the invention patent. It should be pointed out that, for a skilled in the art, several variations and improvements can be made without departing from the concept of the present disclosure, and these all belong to the protection scope of the present disclosure. Therefore, the protection scope of the patent of the present disclosure shall be based on the attached claims, and the description and drawings may be used to interpret the contents of the claims.

## Claims

1. A pharmaceutically acceptable salt of a compound of Formula (I), 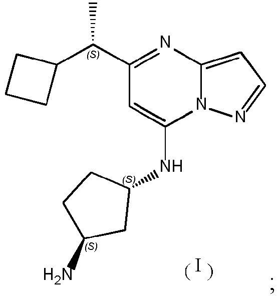 wherein the pharmaceutically acceptable salt is selected from the group consisting of a phosphate and an L-tartrate.

2. The pharmaceutically acceptable salt of the compound of Formula (I) of claim 1, wherein the pharmaceutically acceptable salt is an L-tartrate; and a molar ratio of L-tartaric acid to the compound of Formula (I) in the L-tartrate is (0.8-1.2):1, preferably (0.9-1.1):1, and more preferably 1:1.

3. The pharmaceutically acceptable salt of the compound of Formula (I) of claim 1, wherein the pharmaceutically acceptable salt is a phosphate; and a molar ratio of phosphoric acid to the compound of Formula (I) in the phosphate is (1.8-2.4):1, preferably (1.9-2.3):1, and more preferably 2:1.

4. The pharmaceutically acceptable salt of the compound of Formula (I) of claim 1, wherein the pharmaceutically acceptable salt is a phosphate; and a molar ratio of phosphoric acid to the compound of Formula (I) in the phosphate is (0.8-1.2):1, preferably 1:1.

5. The pharmaceutically acceptable salt of the compound of Formula (I) of claim 2, wherein the L-tartrate is a polymorph, and the polymorph is selected from the group consisting of the following crystal forms:
(1) an L-tartrate crystal form I, having an X-ray powder diffraction pattern with characteristic diffraction peaks at the diffraction angle 2θ (°) values of 13.946±0.2, 16.881±0.2, 19.405±0.2, 21.505±0.2 and 24.262±0.2; and
(2) an L-tartrate crystal form II, having an X-ray powder diffraction pattern with characteristic diffraction peaks at the diffraction angle 2θ (°) values of 11.662±0.2 and 21.353±0.2.

6. The pharmaceutically acceptable salt of the compound of Formula (I) of claim 2, wherein the L-tartrate is a polymorph, and the polymorph is selected from the group consisting of the following crystal forms:
(1) an L-tartrate crystal form I, having an X-ray powder diffraction pattern with characteristic diffraction peaks at the diffraction angle 2θ (°) values of 7.436±0.2, 13.946±0.2, 16.013±0.2, 16.881±0.2, 18.175±0.2, 19.045±0.2, 19.405±0.2, 21.505±0.2, 24.262±0.2, 25.202±0.2, 26.452±0.2, 28.105±0.2 and 31.579±0.2; and
(2) an L-tartrate crystal form II, having an X-ray powder diffraction pattern with characteristic diffraction peaks at the diffraction angle 2θ (°) values of 11.662 ± 0.2, 14.244±0.2, 17.481±0.2, 18.349±0.2, 21.026±0.2 and 21.353±0.2.

7. The pharmaceutically acceptable salt of the compound of Formula (I) of claim 2, wherein the L-tartrate is a polymorph, and the polymorph is selected from the group consisting of the following crystal forms:
(1) an L-tartrate crystal form I, having an X-ray powder diffraction pattern with characteristic diffraction peaks at the diffraction angle 2θ (°) values of 6.687±0.2, 7.436±0.2, 10.615±0.2, 12.053±0.2, 13.164±0.2, 13.946±0.2, 14.875±0.2, 15.201±0.2, 16.013±0.2, 16.881±0.2, 18.175±0.2, 19.045±0.2, 19.405±0.2, 20.659±0.2, 21.505±0.2, 22.434±0.2, 23.04±0.2, 24.262±0.2, 25.202±0.2, 26.452±0.2, 28.105±0.2, 29.692±0.2, 34.139±0.2 and 34.543±0.2; and
(2) an L-tartrate crystal form II, having an X-ray powder diffraction pattern with characteristic diffraction peaks at the diffraction angle 2θ (°) values of 10.376±0.2, 11.662±0.2, 14.244±0.2, 16.548±0.2, 17.481±0.2, 18.349±0.2, 18.984±0.2, 21.026±0.2, 21.353±0.2, 26.925±0.2,29.335±0.2 and 31.784±0.2.

8. The pharmaceutically acceptable salt of the compound of Formula (I) of claim 2, wherein the L-tartrate is a polymorph, and the polymorph is selected from the group consisting of the following crystal forms:
(1) an L-tartrate crystal form I, having an X-ray powder diffraction (XRPD) pattern substantially as shown in FIG. 5; and
(2) an L-tartrate crystal form II, having an X-ray powder diffraction (XRPD) pattern substantially as shown in FIG. 7.

9. The pharmaceutically acceptable salt of the compound of Formula (I) of claim 5, wherein the L-tartrate crystal form I has a thermogravimetric analysis (TGA) spectrum substantially as shown in FIG. 6.

10. The pharmaceutically acceptable salt of the compound of Formula (I) of claim 2, wherein the L-tartrate is amorphous.

11. The pharmaceutically acceptable salt of the compound of Formula (I) of claim 3, wherein the phosphate is a phosphate crystal form I having an X-ray powder diffraction pattern with characteristic diffraction peaks at the diffraction angle 2θ (°) values of 18.234±0.2, 19.131±0.2 and 21.266±0.2.

12. The pharmaceutically acceptable salt of the compound of Formula (I) of claim 3, wherein the phosphate is a phosphate crystal form I having an X-ray powder diffraction pattern with characteristic diffraction peaks at the diffraction angle 2θ (°) values of 13.999±0.2, 18.234±0.2, 18.803±0.2, 19.131±0.2, 21.266±0.2, 22.01±0.2 and 23.247±0.2.

13. The pharmaceutically acceptable salt of the compound of Formula (I) of claim 3, wherein the phosphate is a phosphate crystal form I having an X-ray powder diffraction (XRPD) pattern substantially as shown in FIG. 1.

14. The pharmaceutically acceptable salt of the compound of Formula (I) of claim 11, wherein the phosphate crystal form I has one or more characteristics selected from the group consisting of:
a differential scanning calorimetry (DSC) spectrum substantially as shown in FIG. 2;
a thermogravimetric analysis (TGA) spectrum substantially as shown in FIG. 2; and
a dynamic vapor sorption (DVS) spectrum substantially as shown in FIG. 3.

15. The pharmaceutically acceptable salt of the compound of Formula (I) of claim 4, wherein the phosphate is a phosphate crystal form II having an X-ray powder diffraction pattern with characteristic diffraction peaks at the diffraction angle 2θ (°) values of 18.230±0.2 and 21.144±0.2.

16. The pharmaceutically acceptable salt of the compound of Formula (I) of claim 4, wherein the phosphate is a phosphate crystal form II having an X-ray powder diffraction pattern with characteristic diffraction peaks at the diffraction angle 2θ (°) values of 10.584±0.2, 13.969±0.2, 14.873±0.2, 18.230±0.2, 20.576±0.2, 21.144±0.2, 22.01±0.2, 22.492±0.2, 23.153±0.2 and 23.96±0.2.

17. The pharmaceutically acceptable salt of the compound of Formula (I) of claim 4, wherein the phosphate is a phosphate crystal form II, having an X-ray powder diffraction (XRPD) pattern substantially as shown in FIG. 10.

18. The pharmaceutically acceptable salt of the compound of Formula (I) of any one of claims 15 to 17, wherein the phosphate crystal form II has one or more characteristics selected from the group consisting of:
a melting temperature of 207.48±0.5°C;
a differential scanning calorimetry (DSC) curve substantially as shown in FIG. 11;
a thermogravimetric analysis (TGA) curve substantially as shown in FIG. 11; and
a dynamic vapor sorption (DVS) spectrum substantially as shown in FIG. 12.

19. The pharmaceutically acceptable salt of the compound of Formula (I) of any one of claims 15 to 17, wherein the phosphate crystal form II is anhydrous.

20. A preparation method of an L-tartrate of the compound of Formula (I), comprising the following steps: subjecting the compound of Formula (I) to a salt-forming reaction with L-tartaric acid to form the L-tartrate of the compound of Formula (I).

21. A preparation method of an L-tartrate crystal form I of the compound of Formula (I), comprising the following steps:
subjecting the compound of Formula (I) to a salt-forming reaction with L-tartaric acid in an organic solvent to form a reaction solution; and
slowly cooling the reaction solution to obtain the L-tartrate crystal form I; and
wherein the organic solvent is one or more selected from the group consisting of ethanol, acetonitrile, ethyl acetate, acetone and methanol.

22. A preparation method of an L-tartrate crystal form II of the compound of Formula (I), comprising the following steps:
subjecting the compound of Formula (I) to a salt-forming reaction with L-tartaric acid in an organic solvent to form a reaction solution; and
slowly cooling the reaction solution, and adding an anti-solvent to obtain the L-tartrate crystal form II;
wherein the organic solvent is one or more selected from the group consisting of ethanol, acetonitrile, ethyl acetate, acetone and methanol; and
the anti-solvent is one or more selected from the group consisting of methyl tert-butyl ether, petroleum ether, n-heptane, n-hexane, cyclohexane, isopropanol, acetone, acetonitrile, and ethyl acetate, and the anti-solvent is different from the organic solvent.

23. The preparation method of claim 22, wherein the anti-solvent is one or more selected from the group consisting of methyl tert-butyl ether, n-heptane, isopropanol and acetone, and the anti-solvent is different from the organic solvent.

24. A preparation method of the phosphate of claim 3 or 4, comprising the following steps: subjecting the compound of Formula (I) to a salt-forming reaction with phosphoric acid in the presence of an organic solvent to form the phosphate of the compound of Formula (I).

25. The preparation method of claim 24, wherein, the phosphate of the compound of Formula (I) formed has a molar ratio of the compound of Formula (I) to phosphoric acid of 1:1 or 1:2.

26. The preparation method of claim 24, wherein, the organic solvent is one or more selected from the group consisting of ethanol, ethyl acetate, acetonitrile and acetone.

27. The preparation method of claim 24, wherein, the organic solvent is methanol.

28. A preparation method of a phosphate crystal form II of the compound of Formula (I), comprising the following steps:
subjecting the compound of Formula (I) to a salt-forming reaction with phosphoric acid in an organic solvent to precipitate a solid; and
collecting the solid to obtain the phosphate crystal form II; and
wherein a molar ratio of feed of the compound of Formula (I) to phosphoric acid is 1:(0.8-1.2); the organic solvent is one or more of ethanol, methanol and acetone; preferably, the organic solvent is one or both of ethanol and acetone; preferably, the organic solvent is methanol.

29. The preparation method of claim 28, wherein, the organic solvent is ethanol.

30. The preparation method of claim 29, wherein, the preparation method comprises the following steps:
subjecting an ethanol solution of the compound of Formula (I) to a salt-forming reaction with an ethanol solution of phosphoric acid to precipitate a solid; and
collecting the solid to obtain the phosphate crystal form II; and
wherein the molar ratio of feed of the compound of Formula (I) to phosphoric acid is 1:(0.8-1.2).

31. A preparation method of a phosphate crystal form I of the compound of Formula (I), comprising:
(AI-a) dissolving the compound of Formula (I) in a solvent, adding an aqueous solution of phosphoric acid, and stirring to react; and
(AI-b) cooling a reaction solution obtained in step (AI-a), adding an anti-solvent for crystallization, performing solid-liquid separation, and collecting a solid phase to obtain the phosphate crystal form I of the compound of Formula (I).

32. A pharmaceutical composition, comprising:
(a) the pharmaceutical salt of the compound of Formula (I) of any one of claims 1 to 19; and (b) a pharmaceutically acceptable carrier.

33. Use of the pharmaceutically acceptable salt of the compound of Formula (I) of any one of claims 1 to 19, or the pharmaceutical composition of claim 32 in the manufacture of a medicament for preventing or treating a disease associated with or mediated by CDK9 activity.

34. The use of claim 33, wherein, the disease is one or more of a hyperproliferative disease, a virus-induced infectious disease, and a cardiovascular disease.

35. A method for treating a disease associated with or mediated by CDK9 activity, comprising administering to a subject a therapeutically effective amount of the pharmaceutically acceptable salt of the compound of Formula (I) of any one of claims 1 to 19, or the pharmaceutical composition of claim 32.

36. The pharmaceutically acceptable salt of the compound of Formula (I) of any one of claims 1 to 19, or the pharmaceutical composition of claim 32, for use in preventing or treating a disease associated with or mediated by CDK9 activity.
